(19) Europäisches Patentamt
European Patent Office
Office européen des brevets

(11) **EP 1 785 721 A1**

(12) **EUROPEAN PATENT APPLICATION**

(43) Date of publication:
**16.05.2007 Bulletin 2007/20**

(51) Int Cl.:
*G01N 25/20* (2006.01)    *G01N 33/15* (2006.01)
*G01N 33/48* (2006.01)    *G01K 17/00* (2006.01)

(21) Application number: **05256971.2**

(22) Date of filing: **11.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Stratos Bio Ltd.**
**1205 Geneva (CH)**

(72) Inventor: **Kjeldsen, Naja Joslin**
**4055 Basel (CH)**

(74) Representative: **Benson, John Everett**
**J.A. Kemp & Co.,**
**14 South Square,**
**Gray's Inn**
**London WC1R 5JJ (GB)**

(54) **Microbial, viral and mammalian susceptibility to agents that affect cell growth and metabolism, and mode of action of compounds**

(57) Method of determining the structure activity relationship or mode of action of a first and second compound comprising providing each of the compounds to a microorganism sample and detecting activity of each compound on the sample by detection of the heat generated in the sample using a calorimeter, thereby allowing any difference in activity to be correlated to difference in structure or mode of action between the two compounds.

**Description**

**Field of the Invention**

The present invention relates to use of micro-calorimetry to determine heat output of growing and respiring cell systems (10-100 CFU/ml can be identified) and their susceptibility to active agents which can be determined in vivo. The compatibility between active agent (such as antibiotics, antifungals , antivirals and anti carcinogenic agents) used both alone and in combination can be determined within 1 hour to a variability of less than 5% (which is a measure of high reproducibility) as well as the relationship between cell growth, metabolism and anti-microorganism agents, thus to assess new compounds for safety and efficacy.

**Background of the Invention**

There exists a need to develop more effective diagnostic and therapeutic methods.

**Summary of the Invention**

Microcalorimetric studies of both growing and respiring bacteria and antibiotic action of those have elucidated the mechanism of:

1. Bacterial resistance
2. Emergence of Cancerous Cells
3. AIDS
4. Alzheimer's Disease

Amongst other broad spectrum antibiotics Neomycin and Streptomycin are reported to cause misreading of RNA synthesis. The microcalorimetric results indicate increased protein synthesis of Neomycin treated bacteria followed by increased growth at low concentration of Neomycin. This indicates that the increase in RNA or DNA synthesis depending on whether messenger, ribosomal or transfer RNA is produced acts as a substrate for cell growth (both bacterial and eukaryotic) and viral expression. This increased bacterial growth so called bacterial resistance is an increase due to more nutrients being available to the cells and not a mutation. Increased DNA production and viral expression is seen as a side effect of flagyl metronidazole, commonly used in soft tissue infections and dentistry leading to shingles after treatment, and growth promoting effects in eukaryotes leading to cancer (supported by the NIH JAMA cancer study).

The mechanism of antibiotic or drug induced stimulation of RNA synthesis is as follows:

1. Bacterial resistance Drug induced increased r-RNA synthesis leading to intracellular proteins being produced as nutrients for the bacteria.
2. Cancer: Over treatment by antibiotics leading to increased expression of eukaryotic protein synthesis and thus cancerous growth which is substrate dependent.
3. Increased m RNA synthesis leading to increased DNA synthesis and expression of latent viruses or DNA strands.
4. All of the three above mechanism together in AIDS.
5. Streptomycin induced misreading of RNA synthesis leading to misfolding of

B-pleated sheet in Alzheimers Disease. Evidence:

**1.** Increased respiration and growth of neomycin treated bacteria (Bacillus pumilus and E. coli) visualised by micro-calorimetry (a very sensitive technique that measures heat-output of both respiring and growing cells).
**2.** The use of antibiotics as growth promoting agents in agriculture and a US National Cancer Institute study that has shown the link between breast cancer and antibiotic usage The authors of this JAMA study found that women who took antibiotics for more than 500 days - or had more than 25 prescriptions - over an average period of 17 years had more than twice the risk of breast cancer as women who had not taken any antibiotics.
**3.** The use of antibiotics such as flagyl metronidazole where over use leads to Shingles or expression of latent DNA left over from Chicken pox.
**4.** Duisenberg claimed as early as 1984 that Aids was not a disease but a break down of the immune system by a massive infection. The timing for the emergence of Aids, gay practises ie anal intercourse, drug taking etc and also the observance of increased incidence of tuberculosis which is transmitted by droplet-infection and/or saliva supports this and could also explain why only some people gets active Aids. According to the Global Fund the emerging middle classes of the development countries are the ones contracting Aids and the incidence of the disease is not as high as previously thought. The Foot and Mouth virus of cattle could fall into the same category as AIDS when kept very dirty conditions the cattle contract massive infections of their feet and mouths.

**5.** An Animal case study: The Examples of the present application describe the effect of antibiotic treatment on a cat.

Over prescription of Broad spectrum antibiotics such as streptomycin over decades for non serious infections and the increase in incidence of Alzheimers disease of an ageing population.

Many aspects of the in vivo effects of active agents (such as anti-microorganism compounds) have been insufficiently investigated to date and micro-calorimetry gives us the tools to detect

1. Infection of cells fast and effectively

2. Cell susceptibility to a variety of agents.in vivo

3. Effectiveness of treatment in parallel with the treatment (eg in dentistry, vetenary practices,GP's offices and hospital wards)

4. Emergence of adverse effects such as increase in RNA/DNA synthesis or incompatibility of mixtures of agents or uncontrolled growth of cells

The invention concerns use of micro/nano calorimetry to measure the growth/metabolism of cells (bacterial, viral, mammalian) and interactions of anti-microorganism compounds, including:

Measuring the level of infection of a sample (bacteria, virus, mammalian)

- measuring the interactions between anti-microorganism compounds to determine the compatibility of different compounds when used to together,
- determining the interactions of anti-microorganism compounds with resistant'microorganisms or their susceptibility to RNA synthesis inhibiting agents,
- determining the effect of anti-microorganism compounds on growth/metabolism of cells or microorganisms, and
- determining the effects of compound structure on activity and mode of action.

The invention concerns use of micro/nano calorimetry to measure the interactions of anti-microorganism compounds, including:

- determining the effects of compound structure on activity and mode of action. Accordingly the invention provides a method of determining the structure activity relationship or mode of action of a first and second compound comprising providing each of the compounds to a microorganism sample and detecting activity of each compound on the sample by detection of the heat generated in the sample using a calorimeter, thereby allowing any difference in activity to be correlated to difference in structure or mode of action between the two compounds.

## Detailed description of the invention

Herein the term "cell" is understood to include (bacteria,viruses and mammalian cells) unless the context requires otherwise.

The invention concerns investigating the structure activity relationship or mode of action of compounds. One or more of the compounds which is investigated may act at the cell wall or membrane, or at an intracellular location in the microorganism, such as at a ribosome, or directly on DNA synthesis. In the case where the microorganism is a virus one or more of the compounds may insert themselves into the virus. The mode of action of one or more of the compounds may not be (previously) known. The compounds which are analysed may or may not be related for example to the extent defined herein in the section on related compounds. At least 2, such as at least 3, 4, 5 or more compounds are analysed in the method (by adding them to the same microorganism sample). The compounds may also be tested separately and in any possible combination. In the work described in the Examples of the present application it was found that equimolar amounts gave better biological effects.

In one embodiment the compounds used in the method are not related, and thus typically in the method at least two of the compounds will fulfil one or more of the following criteria when compared:

- They will differ in atomic mass by at least 20, such as at least 30 or at least 40,
- One compound will comprise a ring not present in the other compound,
- One compound will comprise a chain of atoms of a length of at least 5 atoms which is not possessed by the other compound.

The compound may be a biological agent, such as a protein or monoclonal or polyclonal antibody of any origin. The compound may be a nucleic acid. The compound may be a gene fragment. The compound (particularly proteins, peptides and antibodies) may differ in respect of their amino acid sequence, the type or extent of glycosylation (for example differing in the type and number of sugars which are attached) or the type of extent of lipid modification.

In one embodiment the method comprises performing more than one analysis with the compounds, wherein during in each analysis the compounds are added to the microorganism sample in different ratios, which would normally be achieved by providing the compounds in different concentrations (i.e. different molarities). Thus for example in one analysis a first compound will be present at 0.1 to 1% of the molarity of a second compound, or at 1 to 10%, 20 to 30%, 30 to 40 %, 40 to 50 %, 50 to 60%, 60 to 70%, 70 to 80%, 90 to 100%, 100 to 150%, 150 to 250%, 250 to 500%, 500 to 1000%, 1000 to 10,000%. If a first compound is present at a molarity which is 100% of the molarity of the second compound then clearly the compounds are present in a ratio of 1:1 (i.e. an equimolar ratio). Preferably at least 3, such as at least 5, 10, 20, 30 or more analyses are performed with different ratios of the compounds. Typically in the method analyses are performed using at least 2, 3, 5, 10 or more of the specific ratios mentioned above.

In one embodiment the microorganism is pathogenic (for example for any of the species mentioned in the section below on microorganisms, and preferably for humans). The invention relates to a method for determining the structure activity relationship between a first and second compound. In the method the action of each compound on a microorganism is detected by detection of the heat which is generated using a calorimeter. This allows the determination of the structural component present in one of the compounds which causes it to have greater activity than the other compound. The method may comprise analysing a further compound which comprises the structural component found to cause increased activity, but also differs from the first and second compounds used previously. If the further compound is found to have a greater activity than the first or second compound then this allows the identification of further structural components which cause increase in activity.

The compounds which are analysed are typically chemical compounds with amino acid chains attached, biological compounds such as proteins where secondary and tertiary structure will determine biological activity. The compounds may also be related and may for example differ by only a single group, although in certain embodiments they may differ by less than 5, such as less than or 4 or 3 groups. Such groups may be selected from hydrogen, halogen, hydroxy, amino, thio, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ carbocyclyl, 5- to 10-membered heterocyclyl, -Het-L, -L-Het-L', -L-A, -Het-A or -Het-L-A, wherein L and L' are the same or different and represent $C_1$-$C_6$ alkyl or $C_2$-$C_6$ alkenyl, Het is O, S or NR, wherein R is hydrogen or $C_1$-$C_6$ alkyl, and A is a $C_6$-$C_{10}$ aryl, 5- to 10-membered heteroaryl, $C_3$-$C_6$ carbocyclyl or 5- to 10-membered heterocyclyl moiety, wherein the alkyl, alkenyl and alkynyl moieties are unsubstituted or substituted by one, two or three substituents which are the same or different and are selected from halogen, hydroxy, amino and thio substituents and the aryl, heteroaryl, carbocyclyl and heterocyclyl moieties are unsubstituted or substituted by one, two or three unsubstituted substituents selected from halogen, hydroxy, amino, thio, $C_1$-$C_6$ alkyl, $C_2$-$C_6$ alkenyl, $C_1$-$C_6$ alkoxy, $C_1$-$C_6$ alkylthio, $C_1$-$C_6$ haloalkyl, $C_1$-$C_6$ haloalkoxy, $C_1$-$C_6$ haloalkylthio, mono($C_1$-$C_6$ alkyl)amino and di($C_1$-$C_6$ alkyl)amino groups. These can be folded into secondary or tertiary structures and be glycosylated to various degrees.

The invention also provides a method of testing the efficacy of a vaccine in an individual comprising adding in vitro the vaccine to a fluid or tissue sample from the individual and determining whether or not an immune response occurs in the sample to the vaccine by measuring heat output from the sample by a calorimeter.

In a related embodiment the invention provides a method of testing the efficacy of a vaccine in an individual comprising determination of the level and/or type of immune response by measuring heat output from a fluid or tissue sample taken from the individual at a defined time point after administration of the vaccine to the individual, wherein heat output is measured using a calorimeter. The heat output from two or more (such as 3, 4, 5 or more) samples taken at successive time points after said administration may be measured. Thus samples may be taken at one or more of the following time points after administration of vaccine:

- 1 to 6 hours after administration of vaccine, and/or
- 6 to 24 hours after administration of vaccine, and/or
- 1 to 7 days after administration of vaccine, and/or
- 7 days to 1 month after administration of vaccine.

The method may further comprises measuring the levels of antibodies and/or T cells and/or macrophages and/or immune system modulators (such as cytokines) in the sample.

The invention further provides a method of testing whether or not a candidate substance has anti-viral activity comprising adding the candidate substance to a viral sample and detecting interaction of the substance with the virus by measuring the heat output from the sample by calorimetry. Cells capable of being infected by the virus may present in the sample.

The invention includes a method comprising determining whether or not a particular anti-viral compounds or combination of anti-viral compounds is suitable for administering to an individual with a viral condition comprising adding said compound or combination to a fluid or tissue sample from the individual and detecting inhibition of viral activity or growth by measuring heat output from the sample by means of a calorimeter.

The virus may be HIV, HSV, RSV or viruses which are members of the families Picornaviridae (e.g. polioviruses or rhinoviruses); Caliciviradae; Togaviridae (e.g. rubella virus or dengue virus); Flaviviridae; Coronaviridae; Reoviridae (e.g. rotavirus.); Birnaviridae; Rhabodoviridae (e.g. rabies virus); Orthomyxoviridae (e.g. influenza virus types A, B, and C); Filoviridae; Paramyxoviridae (e.g. mumps virus, measles virus, respiratory syncytial virus or parainfluenza virus); Bunyaviridae; Arenaviridae; Retroviradae (e.g. HTLV-I; or HTLV-II; Papillomavirus, the tickboume encephalitis viruses.

The invention provides a method of determining a suitable administration regimens for a patient comprising taking a sample from a patient on a first administration regimen and detecting the effectiveness of the first administration regimen by measurement of the immune response, microorganism load or numbers of malignant cells using calorimetry, and then optionally if the first administration regimen is found to be of low effectiveness selecting a second administration regimen for the patient. The first and second administration regimen may differ in respect to the drug or combination of drugs which are administered. In a preferred embodiment the patient is infected with HIV.

The invention provides a method of determining whether an anti-microorganism or anti-infective compound is able to cause growth of the microorganism/virus/mammalian cell comprising adding the compound to the microorganism, optionally at a pre-determined low concentration threshold, such as < 10xMIC, <8xMIC, < 2xMIC, < 1xMIC to determine whether or not growth or heat output of the microorganism/virus/mammalian cell occurs in response to the addition of the compound, wherein growth of the microorganism is optionally measured by detecting heat output from the microorganism/virus/mammalian cell using a calorimeter, and wherein the compound may be alone or in a combination with 2 or more other anti-microorganism or anti-infective agents. The threshold may be less than 0.01ug/ml, 0.01 ug/ml to 10ug/ml, such as 0.1 ug/ml to 1ug/ml.

If the said compound is found to cause increased growth of microorganisms at the pre-determined low concentration, then the compound may be administered to an individual suffering from a condition caused by the microorganism or at risk of such a condition, at a high dosage, but optionally not at dosages causing increased respiration of the said compound

*Samples for use in the embodiments described herein*

The sample is typically urine, stools, whole blood, plasma, a body secretion (or a swab thereof) or a tissue sample. The sample may or may not be processed before it is analysed in the method of the invention, but in a preferred embodiment it is not processed before analysis. Such processing typically results in a removal of a component from the sample or may result in dilution of the sample (such as dilution of a swab sample). As discussed below substances such as nutrients may be added to the sample to aid growth of the microorganism. Such additions of substances are not understood herein as being "processing" of the sample.

Typically the sample (at the point of being taken from the individual) has a volume of less than 10 millilitres, such as less than 5, less than 3 or less than 3 millilitres, down to for example 25 ul or less. The sample which is analysed by calorimetry typically has a volume of less than 10 millilitres, such as less than 5, less than 3 or less than 3 millilitres, down to for example 25 ul or less.

The individual on whom diagnosis is performed may be from any species, and is typically a bird or mammal. The individual is preferably human. The individual may be one which is in a state that renders it susceptible to infection by the microorganism, or susceptible to cancer.

*Microorganisms and cells which are used and/or detected in the methods of the invention*

Any suitable microorganism may be used or detected in the methods of the invention. The microorganism is typically a bacterium, yeast, fungus or protozoa or virus. A tumor or malignant cell culture may be used or detected in the method of the invention.

The microorganism may be vancomycin resistant enterococci, pencillin resistant Streptococcus pneumoniae, methicillin resistant Staphylococcus aureus, multi-drug resistant gram-negative bacillus, multi-drug resistant Mycobacterium tuberculosis, multi-drug resistant gram-positive bacteria, Achromobacter, acinetobacter, aerococcus, aeromonas, alicalignes, bacillus, bacteroides, burkholderia, chryseobacterium, citrobacter, clostridium, corynebacterium, enterobacter, enterococcus, escerichia, fusobacterium, haemophilus, hafnia, klebsiella, listeria, micrococcus, moraxella, morganella, pantoea, peptococcus, peptostreptococcus, proteus, providencia, pseudomonas, salmonella, serratia, staphylloccocus,stenotrophomonas, streptococcus multi-drug resistant malaria parasite or HIV, HSV, RSV or viruses which are members of the families Picornaviridae (e.g. polioviruses or rhinoviruses); Caliciviridae; Togaviridae (e.g. rubella virus or dengue virus); Flaviviridae; Coronaviridae; Reoviridae (e.g. rotavirus.); Birnaviridae; Rhabodoviridae (e.g. rabies virus); Orthomyxoviridae (e.g. influenza virus types A, B and C); Filoviridae; Paramyxoviridae (e.g. mumps virus, measles virus, respiratory syncytial virus or parainfluenza virus); Bunyaviridae; Arenaviridae; Retroviradae (e.g. HTLV-I ; or HTLV-II); Papillomavirus, or a tickboume encephalitis virus.

*Conditions under which detection is performed*

In the method of determining a structure activity relationship (and thus mode of action) the relevant compound (s) are simply contacted with the microorganism and the heat generated is detected. No further components need to be added to microorganism sample, and thus for example the microorganism does not need to be in conditions in which growth will occur (as respiration studies can also be used to detect susceptibility to a compound depending on the mode of action of the compound).

Typically detection of heat is carried out for at least 2 to 48 hours, such as 3 to 24 or 8 to 12 or 4 to 5 hours after addition of the compound(s). Preferably continuous measurement of heat detection is performed, such as at least once every 10 minutes, such as over any of the above specified time periods. For example over a 24 or 48 hour period cell death can be demonstrated (and thus the bacteriocidal effect of antibiotic in vivo), or over a 2 hour period inhibition of growth (and return to baseline) or compatibility of compounds.

In the method of diagnosis the sample from the individual is placed in conditions which allow growth of the microorganism to be detected. Thus typically nutrients are added to the sample which aid growth of the microorganism. In the case where the sample which is analysed has more than one microorganism the medium which is used may favour the growth of one microorganism and not the other. The medium may be a defined medium, blood broth, nutrient broth, a defined medium for a known microorganism, cell culture medium or a saline solution (for respiratory studies).

The nutrients may be species specific, i.e. aiding the growth of some species of microorganism more than others. This may assist in allowing determination of the species of microorganism causing the infection.

The sample may be placed in a temperature which allows growth/heat output of the cell or microorganism to be detected, such as in a temperature of 20 to 45 degrees C, typically in a temperature of 30 to 40 degrees C, such as at a temperature of 35 to 38 degrees C or at 37 degrees C.

The sample is typically placed in conditions allowing growth of the cell or microorganism for 3 to 12 hours, and typically for less than 8 hours, such as less than 6 or less than 4 hours.

As mentioned above in the method heat produced by growth of the microorganisms is detected. In one embodiment the heat produced is analysed over time, for example in the form of a graph of heat produced versus time (or power versus time). Such an analysis may allow detection of the quantity of microorganisms present in the original sample (thus giving an indication of the severity of sepsis) or may allow determination of the species of the microorganism. The heat data may be compared to control data to aid in ascertaining microorganims numbers and/or species.

The micro/nano calorimeter which is used in the method of the invention is normally one which is capable of continuously measuring heat effects (enthalpy changes) of a sample. In one embodiment the calorimeter is capable of detecting the heat output of $10^2$ to $10^5$ CFU/ml of microorganisms, such as of any of the species mentioned herein. Typically the calorimeter is capable of detecting a rate of heat change of 1uW or 1nW. The calorimeter may be capable of measuring heat output simultaneously from more than one sample, such as from at least 4, at least 20, at least 50 or at least 100 samples. During analysis of the heat output the sample may be in aerobic or anaerobic conditions.

The invention also provides a kit for carrying out a method according to any of the preceding claims comprising a calorimeter suitable for use in said method. The calorimeter may be specifically adapted so that any of the types of samples mentioned herein can be analysed and/or data concerning heat output from a sample can be provided to a computer or database for analysis of the data.

*Growth caused by anti-microorganism compounds*

One aspect of the invention is based on the effect of low concentrations of anti-microorganism compounds on the growth of cells. Such cells can be bacterial cells (such as an any type or species of microorganism mentioned herein), preferably bacterial cells, or eukaryotic cells. It has been found that certain anti-microooganism compounds, especially compounds that have an effect on RNA synthesis, can cause cells to grow and thus may explain bacterial resistance (increased intracellular protein synthesis providing nutrients for the bacteria in vivo), viral infections (increased synthesis of mRNA and the expression of dormant DNA fragments, e.g. the appearance of shingles after administration of antibiotics) and also the emergence of HIV (a mixture of the two above mechanisms eventually rendering the immune system ineffective). The same effect in eukaryotic cells would give rise to tumour growth. Thus previously observed apparent resistance to anti-microorganism compounds may be due to stimulation of growth of the relevant microorganism at a concentration of anti-microorganism compound which is too low or alternatively due to stimulation of eukaryotic growth when administered in large excess or when no microorganism is present.

The effect of the anti-microorganism compound on cells is consistent with the known growth promoting effects of antibiotics. This also suggests a mechanism for anti-microorganism compounds to cause cancer.

The present work thus provides evidence of actions of anti-microorganism compounds which can cause detrimental effects in the individual being treated. Such actions will contribute for example to some of the side effects observed when using anti-infective compounds.

Accordingly the invention provides a method of determining whether an anti-microorganism compound (e.g. an anti-infective compound) is able to cause increased growth or respiration of the microorganism/virus/cell comprising adding the compound to either a respiring or growing microorganism, optionally at a pre-determined low concentration threshold, such as < 10 X MIC, <8 X MIC, < 2 X MIC, or < 1 X MIC. The threshold may be 0.01 ug/ml to 10ug/ml, such as 0.1 ug/ml to 1ug/ml. A single anti-microorganism compound may be added or 2, 3, 4 or more anti-microorganism compounds may be added.

For neomycin (with a known mode of action of cause misreading during RNA synthesis) an increased heat output can be observed at concentrations of < 1ug/ml or in combinations with other antibiotics. For respiring E.coli all concentrations of Neomycin caused an increased heat output.

This can be used to determine whether or not growth of a microorganism/virus or cell occurs in response to the addition of the compound. Preferably growth of the microorganism/virus or cell is measured by detecting heat output from the microorganism/virus or cell using a calorimeter, such as in any of the ways discussed previously. The microorganism is generally any microorganism that causes disease, for example any such microorganism disclosed herein.

If the said compound is found to cause increased growth of microorganisms at the pre-determined low concentration, then appropriate action can be taken in the manner in which it is used, for example it can be administered to an individual suffering from a condition caused by the microorganism or at risk of such a condition, at a high dosage which would ensure killing of the microorganism. It can also be used to monitor infections in patients and thus administer anti-infective agents when necessary and stop treatment when the microorganism/virus or cell is dead (indicated by return to baseline of heat output, confirmed by viable count measurements).

*Anti-microorganism compounds*

The anti-microorganism compound may be Ampicillin, Chloramphenicol, Erythromycin, Nalidixic acid, Tetracycline, Aminoglycoside (such as streptomycin, kanamycin, gentamicin, tobramycin, amikacin, netilmicin and neomycin), tetracycline, minocycline and doxycycline, Spectinomycin, lincomycin, clindamycin, a macrolide (such as Erythromycin, clarithromycin, azithromycin or spiramycin), Fusidic acid, Rifampin, rifamycin, rifampicin, Quinolone (such as nalidixic acid, ciprofloxacin, oxolinic acid, Ofloxacin, norfloxacin, levofloxacin, lomefloxacin, sparfloxacin), sulfonamide, sulfone, Trimethoprim, methotrexate, pyrimethamine, Para-aminosalicylic acid (PSA), Dapsone, Isoniazid (INH), Spectinomycin, a Penicillin, flagyl metronidazole, Actinomycin, a sulphadrug, vancomycin, adrenamycin, daqucomycin, rifamycin, heparin, afo/3, streptoygidin, or Streptovaricin.

*Administration of agents*

As mentioned above the invention includes administration of particular agents. Such an agent will generally need to be formulated into an appropriate composition to make it suitable for administration. The agent will typically be administered to an individual (human or animal) in need thereof.

The formulation will depend upon factors such as the nature of the substance and the individual to be treated. Any such substance may be administered in a variety of dosage forms. It may be administered orally, parenterally, subcutaneously, intravenously, intramuscularly, intrasternally, transdermally or by infusion techniques. The substance may also be administered as suppositories.

Typically the substance is formulated for use with a pharmaceutically acceptable carrier or diluent. The pharmaceutical carrier or diluent may be, for example, an isotonic solution.

An effective amount of substance is administered. The dose may be determined according to various parameters, especially according to the substance used; the age, weight and condition of the individual to be treated; the route of administration; and the required regimen. A typical daily dose is from about 0.01 to 500 mg per kg, preferably from about 0.1mg/kg to 10mg/kg of body weight.

The following Examples illustrate the invention:

Examples

A micro/nano calorimeter is an apparatus that can measure the heat lost or gained during all chemical, physical and biological processes. The heat produced during these processes creates a temperature difference between the sample in the test chamber and its surroundings. The intensity of the process can thus be established through continuous monitoring of the temperature difference. The instrument used in the work is a heat conduction microcalorimeter and is designed to minimise temperature difference and encourage the most rapid heat flow away from the reaction site to a

large heat sink ($\Delta T < 10^{-4}$ °C).

Micro/nano calorimetry is a non-destructive and non-invasive direct technique that does not require any prior sample treatment (like the lysis-filtration with impedance detection method). The analysis is also not limited to a physical state of a sample i.e. the calorimeter can deal with any sample form: solids, liquids, gases, transparent and non-transparent samples alike can be analysed. The colour of the sample is also not an issue using calorimetry. The calorimeter's indifference to colour and substrate nature makes it an ideal system for analysing the red, complex, suspended, cell composition of blood. Calorimetry provides a favourable advantage to the classical method of blood culturing as it can analyse very small samples of blood.

If an uninfected sample is placed in a calorimeter, one would expect there to be a clinically insignificant number of organisms present per ml (i.e. <$10^4$ organisms/ml). It would thus take some time for the organisms in the sample to grow (to >$10^4$ organisms/ml) and produce enough heat so that it can be detected in the calorimeter. Conversely, an infected sample will have >$10^4$ organisms/ml. When this sample is placed in a calorimeter, it would have enough organisms to generate an immediate detectable level of heat.

Media Dependency

The media employed in classical microbiological assays are almost invariably complex, containing such constituents as beef extract, yeast extract, blood components and peptone. Such media may not be the optimal choice for a microbiological assay.

Constituents of complex media, such as yeast extract and peptone, will vary from batch to batch; even different samples of a complex medium, derived from the same batch of its constituents, may be different after autoclaving. The response of a culture to small changes in medium composition could be a serious limitation to any microcalorimetry assay; hence, a complex medium may not be the optimal choice.

To obtain defined, reproducible media, synthetic media of known chemical composition are better.

The pH of the medium should be constant throughout incubation. Fluctuation of pH will have the following effects: (i) metabolism may vary as a function of pH, (ii) the heat associated with the removal of substrates and the appearance of metabolites will depend upon their respective degrees of protonation, and (iii) the activity of candidate substance which is being tested may vary as a function of pH.

Antibiotics are known to combine with several medium components. For example, binding to proteins and salts is common and due to these effects, the active concentration of the antibiotic is often less than calculated. In addition components of the media may affect the death rate or rescue treated cells and/or inhibit their interaction with the antibiotic. Using a defined medium, the effect of medium components on antibiotic action can be investigated and controlled.

Importance of a Reproducible Inoculum

Liquid nitrogen refrigeration (-196°C) may be used in the preservation of microorganisms. Inocula stored at this temperature are superior to those stored at higher temperatures in that they retain constant viability over long period.

In addition to long time viability inocula recovered from storage in liquid nitrogen give standard inhibition zones in plate assays and standard turbidity for a standard dose of antibiotic. More importantly consistent electron micrographs showed no damage of the cell wall or membrane on freezing and thawing. Consistent electron micrographs also showed the anticipated effects of antibiotics on cells given their mode of action.

Importance of Oxygen Tension

Oxygen tension is a crucial factor in the growth of facultative anaerobes and aerobes. The oxygen available for microbial growth is dependent on oxygen solution rate and this is dependent on fermentor conditions e.g. size, volume of medium, flushing rate, stirring rate and number of baffles. Since the oxygen tension is influenced by so many factors which all have to be accurately reproduced for reproducible assays it is better to adopt strict aerobic or anaerobic conditions.

Organisms

All bacterial cell preparations were suspended in Ringers solution or 10% DMSO and frozen and stored under liquid nitrogen. The viable count of thawed inocula was determined using the surface spread plate method.

The Calorimetric Medium

a) For B. pumilus a simple glucose/salts medium was employed with a glucose concentration of 0.1 % w/v.

b) For Bord. bronch. The above salts medium was used with the addition of nicotinic acid, methionine and glutamic acid. Glutamic acid was used at a concentration of 0.2% w/v.

c) For M. flavus a semidefined medium was employed, using the same salts as a B. pumilus but also Na-citrate, biotin and protease peptone. In the calorimeter a concentration of 0.4% protease peptone was used. All medium constituents (except protease) were analytical reagents. The calorimetric medium was therefore a final concentration of 100ml of either solution a, b or c freshly autoclaved for 15 mins exactly at 121°C, 151b/sq inch. Glucose was added to the appropriate solution after autoclaving.

The Calorimetric Incubation Vessel

The bacterial cultures were grown in a fermenter vessel of approximately 200ml capacity which had a flat ground-glass rim. To the vertical face of the vessel close to its base, equidistantly spaced around the circumference, ports were fitted for an oxygen electrode and for sampling/inoculation. The fermenter was closed with a flat flanged glass lid which had five ports accommodating respectively, gas inlet tube, gas exhaust (via air condenser), inlet and outlet flow-lines to and from the calorimeter. The fermenter and lid was sealed with a p.t.f.e. gasket and clamped with a metal clip. The fermenter was placed on a magnetic stirring unit in a water jacket which allowed circulation of the thermostatically controlled water at 30°C or 37°C. The fermenter which contained a Teflon coated magnetic stirring bar, was sterilised by autoclaving.

The Calorimetric Incubation

For a typical calorimetric incubation the medium was passed through the calorimetric vessel at the same flow rate and temperature as those used in the experiment to establish a steady baseline deflection. The following operations then took place to a strict timetable. The liquid nitrogen stored ampoule was thawed at 38°C for 3 min and then stirred for 20 sec on a whirlimixer. The bacterial suspension (0.5-1.5ml) volumes was inoculated into the medium (100ml) two minutes after completion of thawing. A Gilson automatic pipette was used for inoculation. The calorimeter inlet flow-line was placed in the incubation medium 30 seconds after inoculation. The flowing incubation medium reached the calorimetric vessel two min after inlet flow-line insertion. At this time the recording of the p-t curves was started. Eight min after inoculation the outflow from the calorimeter was returned to the fermenter (after the "baseline" medium had run to waste but before the inoculated medium ran to waste) thus making a closed system. Aeration was started as soon as the inlet tube was inserted in the fermenter. Microbiological purity of the culture was checked by plating out a sample of the culture at the end of each incubation on nutrient agar (lab M).

The flow-microcalorimeter cannot be sterilised by conventional methods but can be decontaminated by successively pumping through solutions of 0.1 % w/v NaOH and finally sterilised deionised water. (For M. Flavus it was necessary also to use 70% w/v ethanol as well to remove any traces of peptone in the flow lines).

Presentation of Calorimetric Results

The output of calorimetric experiments is a plot of power (energy evolution per unit of time) as a function of time. The unit of power (p) is the Watt (W) and the unit of time is the hour (h). In some experiments seconds (s) were used as time unit.

Growth Estimation

Dry Weight

Cell suspensions were centrifuged on a MSE bench centrifuge at 4000 rpm, washed twice and resuspended in ¼ Ringers and then washed and resuspended in a small amount of distilled $H_2O$. This suspension was dried in an oven at 115°C for 24 hours (to a constant weight). For the preparation of a standard curve of dry weight versus optical density, optical densities of varying dilutions of the cell suspension in Ringers solution were determined and a standard curve constructed. This was related to dry weight as 100% being the suspension before dilution and the dry weight determined relative to this figure.

Viable Counts

Two methods of viable counts were used a) surface drop plate method and b) spread plate method.

a) Surface Drop Plate Method

Petri dishes containing ca 20ml of agar (lab M nutrient both 25g, lab M agar 10g, glass distilled water to 11) were prepared in advance and dried by incubation at 37°C. Serial tenfold dilutions were made in ¼ strength Ringers of 10% DMSO solution and 10 replicate drops 0.02ml volume (bacteriological dropping pipette, Astell) of appropriate dilutions were delivered onto the surface of each plate. After incubation from 18-28h at 37°C (depending on the bacterial culture) counts were determined from the drop areas that contained the highest number of colonies (5-50) and which were not affected by confluence or overcrowding. The viable count was expressed as colony forming units (cfu) per ml. The 95% confidence limits of the viable counts were obtained using the following expression:

$$\left(Cm + 1.96\sqrt{Cm}\right)\ \left(Cm - 1.96\sqrt{Cm}\right)$$ where $Cm$ is the total number of colonies counted on $C$ plates and $m$ is the mean number per plate. Consequently the 95% confidence limits for the observed mean per plate ($m$) are:

$$\frac{Cm + 1.96\sqrt{Cm}}{C} \qquad \text{and} \qquad \frac{Cm - 1.96\sqrt{Cm}}{C}$$

b) Spread Plate Method

Plates and tenfold serial dilutions of cell suspension were prepared as described above. An aliquot of 0.1ml of each of four successive dilutions was spread on each of five plates using an alcohol sterilised glass spreader. Colonies were counted after incubation of (18h-28h) at 30°C and counts ere expressed as cfu/ml. 95% confidence limits were calculated as described above.

Spectrophotometric Measurements of Growth

Cell Density was measured spectrophotometrically by determining the optical density of cell suspensions against suitable blanks at 540nm (green filter, pathlength 1cm) with a Corning-Eel spectrophotometer. These values were converted to dry weights using a curve of OD against dry weight.

Liquid Nitrogen Storage

The optimum cooling velocity is may be 0.5-10'C per min for most biological cells through a critical freezing range from ambient temperature to -50°C. Rapid thawing may lead to the highest recovered viability of cells.
The application of liquid nitrogen frozen inocula to bioassays has been strictly limited. The lack of acceptance can be attributed to the essentially empirical nature of freezing techniques and the failure to discriminate low temperature storage (-160 - -195°C) from storage at temperatures only slightly below ice temperature (-4 - - 20°C).

Bacterial Cultures

Cryobiology is so complex that no firm guidelines or predictions can be made regarding the successful freezing and recovery of a particular strain. The problem is made more difficult because the detail in most reported studies is not sufficiently complete to determine the basic factors that were responsible for cell death. The cooling and thawing rates used are often imprecisely described and little attention is given to such details as strain, culture conditions and suspending medium. The main causes for this poor documentation are (i) that many investigators are merely concerned with the pragmatic objective of ensuring culture survival even when this is extremely low and (ii) the great diversity of application of frozen cultures. Consequently information regarding the mechanism of cell injury has, in the main, been a secondary consideration.

Cryoprotectants

Cryoprotectants reduce the amount of ice and thus reduce the build up of ice in the cell by shrinking the cell before cooling (if they are non permeant), or providing osmotic fluxes during and after thawing as they leave the cells (if they are permeant). There is evidence that during freezing and thawing normally excluded solutes (e.g. sucrose for erythrocytes) can enter the cells. It is also possible, that cryoprotectants may have a direct action on cell membranes, that alters the response of the membrane to stress.

Any solute that can be elaborated in or penetrate cells will lower the vapour pressure of the aqueous solution and reduce the amount of ice formed at any temperature. The latter is termed the colligative mechanism. Colligative cryoprotection can be achieved by glycerol or DMSO for example. This type of protectant must be non-toxic to the cells in the high concentrations (2-4M) required, and protect the cells against injury from slow freezing.

Cryoprotection could also be afforded by prevention or reversal of hypertonic injury. This is the probably function of so called extracellular cryoprotectants (e.g. PVP and hydroxyethyl starch). Also capable of some cryoprotection are sugars and sugar alcohols. Unlike colligative agents, most non penetrating cryoprotectants are effective in very low molar concentrations.

One consistent characteristic of non penetrating cryoprotectants is that, to be effective, they must be used in conjunction with relatively rapid freezing and rapid thawing. Rapid freezing and thawing serves to minimize solution effect injury by limiting the time available for its development.

Rate of Freezing and Thawing

A guideline to freezing and thawing rates is as follows:

slow freezing: 1 to 20°C/min
fast freezing: fall in temperature of 170-200°C/min
ultra fast freezing: (droplet) fall in temperature of 100°C/sec
slow thawing: rise in temperature of 20°C/min

Development of a Defined Medium

The growth characteristics of B. pumilus, M. flavus and Bord. bronch were examined.

The criteria for development of media were as follows:

a) simplicity and definition of medium
b) length of lagtime
c) growth rate
d) uniformity of growth

Growth Curves

Bacillus pumilus

B. pumilus was grown in a batch culture (25/50ml), shaken at 200 rev/min on a rotary shaking incubator at both 30 and 37°C in 250ml conical flasks fitted with a side arm (test tube) so that growth could be followed turbidimetrically using an EEL-spectrophotometer (green filter, 540nm) with distilled water as reference. Bacteria were inoculated form an overnight culture grown in a defined medium with varying glucose concentration (0.05% w/v - 1% w/v).

Medium constituents (g/l deionised $H_2O$):

| pH 7.9 | pH7.0 |
| --- | --- |
| $KH_2 PO_4$ 0.72g | $KH_2 PO_4$ 8.75g |
| $K_2HPO_4$ 13.58g | $K_2HPO_4$ 3.75g |
| $(NH_4)_2 SO_4$ 2g | $(NH_4)_2 SO_4$ 2g |
| $MgSO_4$x $7H_2O$ 0.125g | $MgSO_4$x $7H_2O$ 0.125g |
| trace elements 1ml | |

Trace elements: g/l deionised $H_2O$

MgO: 10.75g, CaCo3: 2.0g, $FeSO_4$x $7H_2O$: 4.5g,
$ZnSO_4$x $7H_2O$: 1.44g, $MnSO_4$x $4H_2O$: 1.12g
$CuSO_4$x $5H_2O$: 0.25g, $CoSO_4$x $7H_2O$: 0.28g

H₃BO₃: 0.06g and concentrated HC1: 51.3 ml.

Finally pH 7.9 and 30°C were settled on as pH 7.9 as this is the optimum activity for neomycin and the bacteria aggregated at 37°C.

Bord. bronch. was grown with the same equipment as B. pumilus. The temperature optimum was given in Bergeys manual as 35-37°C and pH 7, the following simple medium was designed: Salts: as B. pumilus pH 7 and a requirement of 1-μg/ml methionine and 1-μg/ml nicotinic acid. As carbon source glutamic acid was used. (0.05-1%) Trace elements as B. pumilus.

Micrococcus flavus growth technique and equipment was the same as describe above.

Salts: as B. pumilus at pH 7.00 with the addition of 0.125g sodium citrate per 1 plus biotin 25μg/ml and protease peptone) Oxoid) at various concentrations ranging from (0.1% w/v- 1% w/v). Trace elements as B. pumilus. pH7 was chosen as the pH of optimum binding for bacitracin and 30°C as the optimum growth temperature for M. flavus.

All chemicals used in this project were of analytical grade. All media were freshly made up and sterilized by autoclaving for 15 min exactly at 121 °C, 15 pounds/sq inch.

Organisms

Bacillus. pumilus

B. pumilus was obtained as a spore suspension from ICI Macclesfield and maintained on agar slopes (25g nutrient broth lab M, 20g agar lab M/1 glass distilled H₂O). Stock cultures were subcultured every six months and stored at 4°C after incubation at 37°C for 16-26 hours. Working cultures were subcultured from the stock cultures when needed.

Bordetella bronchiseptica

Bord. Bronch: NCTC 8344 was obtained from ICI, Macclesfield and maintained as B. pumilus except that it was grown at 37°C.

Micrococcus flavus

M. flavus NCTC 7743 was obtained from ICI, Macclesfield. It was kept as described for B. pumilus.

Standard Growth Regimen

All the media were as described all stock solutions were made up as follows: phosphate buffer 5x required strength, salts for B. pumilus and M. flavus 5x required strength; biotin solution 100x required strength, glucose and glutamic acid 2.5% w/v. the medium constituents for Bord. bronch. were made up to 2x required strength (excluding glutamic acid and buffers).

All these constituents were mixed and then autoclaved at 121°C (15 pounds/sq inch) for 15 minutes exactly. Glucose was added aseptically after sterilisation.

The growth medium for freezing was prepared as follows:

18 sterile 500ml baffled conical flasks (containing 100ml of the medium in question) were inoculated with an overnight culture (in the same medium) of the organism to be frozen. The growth curve was followed using conical flasks with side arms. The organisms were harvested in late logarithmic phase and stored overnight at a constant temperature of 4°C. No deterioration in viable counts was detected after overnight storage for any of the bacteria in question. The bacteria were then harvested by centrifugation (3500 rev/min) for 15 minutes using a MSE bench centrifuge. The combined bacterial pellets were washed once with Ringers solution (NaCl 25g, KCl 11g, CaCl₂ 0.75g/1 deionised H₂O) and then suspended in either Ringers or 10% w/v DMSO (Bord. bronch. only).

Freezing of Bacterial Suspensions

The cell suspension was dispensed in 2ml aliquots in 12x35mm sterile polypropylene ampoules (Sterilin Ltd). The cells in the reservoir were continuously stirred to facilitate uniform dispensing of the inocula. Charged ampoules were capped immediately after filling an held onto a bar by uniformly spaced terry clips (1.2cm diameter) which gripped the caps. Liquid nitrogen (initially 10 1) was contained in an expanded polystyrene (2.5cm) insulated aluminium tank (51x30x15cm). The loaded bars were supported on the sides of the tank, so that the ampoules were exposed to the liquid nitrogen vapour but not immersed or even touching the liquid nitrogen. There was approximately 10 cm between the surface of the liquid nitrogen and the bottom of the ampoules at initiation of cooling. With technical assistance 200 ampoules can be dispensed and loaded onto the cooling bars within 20 min. The ampoules were released from the terry clips and plunged into liquid nitrogen over a period of 20 sec after the temperature of the bacterial suspension had passed -80°C. A cooling curve was recorded for each freeze using a separate sample in contact with an alcohol thermometer (-100 - +30°C). the ampoules were removed from the freezing nitrogen cryostat (CV 35, British Oxygen Company).

Recovery of Ampoules

Ampoules were recovered by removal from the cryostat using pre-cooled forceps, and placing in a water bath at 38°C for 3 min without agitation before being removed to room temperature.

Determination of Viability of Bacterial Cultures

The viability of the pre-freeze and post-freeze suspensions was determined by the spread plate method. Dilutions of bacterial suspensions were carried out in either Ringers solution or 10% DMSO, depending on the nature of suspension used for freezing.

DEVELOPMENT OF A DEFINED MEDIUM

The development of a defined medium for Bord. bronch. was carried out to ensure maximum control of the factors determining microcalorimetric P-t curves. Initially the bacteria were grown in complex medium containing salts and 0.1% or 0.2% protease or 0.2% protease + 0.2% glucose or 0.2% protease + 0.05% yeast extract. The initial pH was 7.00. The medium was inoculated form an overnight culture grown on salts plus 0.2% protease medium and incubated at 37°C. After 16h the yield was significantly higher in the medium that contained yeast extract. This could be due to some essential vitamin being present in the yeast extract. According to Bergeys manual Board. bronch. requires nicotinic acid which is present in yeast extract. A defined medium has been developed for Bord. pertussis that contains proline, cysteine and glutamic acid as carbon and nitrogen sources, various salts, Tris buffer, ascorbic acid, nicotinic acid and glutathione. However growth of more 0.3mg dry weight/ml was not achieved in this medium with the strain of Bord. bronch. used here. The medium was modified by using proline and glutamic acid at various concentrations up to 0.4% both with and without 0.2% glucose. However growth did not exceed the above limit even when the cultures were incubated at 37°C for four days.
Therefore it was decided to investigate a medium consisting of salts, trace elements, methionine, nicotinic acid, proline, glutamic acid and glucose. Methionine and nicotinic acid were at concentrations sufficient to meet specific growth factor requirements. Proline, glutamic acid and glucose at levels sufficient to act as carbon and energy sources and the amino acids possibly also as nitrogen sources. Salts and trace elements were at concentrations required by mocroorganisms in general. Citrate as metal chelating agent was not used as Bord. bronch. is reported to utilise citrate.
Good growth could be obtained with glutamic acid plus methionine and nicotinic acid as the only organic constituents. The presence of nicotinic acid (1 $\mu$g/ml) was essential to good growth but could be added as yeast extract. Growth was poor with glucose plus methionine and nicotinic acid, but high yields were obtained when glucose was replaced by glutamic acid or proline. No improvement in yield was achieved by using proline and glutamic acid or by adding glucose to proline or glutamic acid containing medium.
The doubling times were roughly the same in all media containing proline and or glutamic acid plus methionine and nicotinic acid, but the lag time was shortest (25 min) for a medium containing both glutamic acid and proline. However the lag time for a glutamic acid medium without either proline or glucose was 40 min. As the objective was to design the simplest possible medium it was decided to use glutamic acid as the source of carbon, energy and possibly nitrogen together with a mixture of salts, methionine and nicotinic acid. A series of experiments with media containing different glutamic acid concentrations was then carried out.
The doubling times were roughly the same for all concentrations of glutamic acid but when methionine was excluded from the medium the doubling time increased considerably. Lag times varied little with glutamic acid concentration but glucose seemed to increase lag time at the lower concentrations of glutamic acid. Glutamic acid was utilised fully for all concentrations assayed except when the medium did not contain methionine and when glucose was added.
At a glutamic acid concentration of 0.05% with addition of 0.1% glucose no glucose was utilised. When 0.2% glucose was added to 0.2% glutamic acid 75% of the glutamic acid was utilised and half the glucose. Little difference in final yield was observed with and without glucose. When dry weight against 0D and dry weight against glutamic acid concentration was analysed it was found that the relationship between yield and glutamic acid concentration is linear up to 0.1% glutamic acid. It was concluded that 0.2% glutamic acid would probably be suitable concentration for the calorimetric incubations because of the relatively short lag and good doubling times. At higher concentrations of glutamic acid pH increased considerably during growth.

Analyses of P-T Curves

The p-t curves obtained by growing Bord. bronch. in various concentrations of glutamic acid all have the same general appearance. A small peak after 6h 30min and a broad peak after approximately ten hours. The small peak suggests some regular change in metabolism e.g. change in oxygen tension, in toxin production or in C or N source. This change in power, was quantitative and reproducible. The peak height increased with increased glutamic acid concentration although at an initial concentration of 0.2% glutamic acid no change could be detected in glutamic acid concentration after 7h. The peak height varied from 20-40% of full scale deflection when increasing glutamic acid concentration from 0.1 % to 0.2% and again to 80% of full scale deflection when using 0.3% glutamic acid. This was also true for the broad peak although the power increase from 0.2-0.3% glutamic acid was only 40%.

Very little variation in the slope of the small peak at different concentrations of glutamic acid could be observed - suggesting that the kinetics of metabolism were the same for different concentrations of glutamic acid.

To be able to distinguish the small peak at all is a great improvement in the sensitivity of the calorimeters and the reproducibility of our assay procedure. Very few workers have achieved a greater sensitivity than that of 100$\mu$V full scale deflection which is required to distinguish the small peak for Bord. bronch. Differences in p-t curves of different bacteria can be used as a means of identifying them, but differences in p-t curves were usually media dependent and when media constituents were changed completely different p-t curves arose. These changes were indicative of changes in metabolism which were reproducible and quantative.

Freezing Procedure

The procedure for growth of B. pumilus for freezing was that described previously. To achieve maximum yield for freezing the bacteria were grown in a 0.8% glucose/salts medium. The bacteria were harvested towards the end of log phase, yield, 1.13 mg/ml dry weight and treated as described. B. pumilus was difficult to resuspend after centrifugation as aggregation occurred. This was avoided, to some extent, by growing the bacterium in baffled flasks and resuspension in Ringers solution rather than DMSO. The bacterium was frozen down as described above.

The freezing curve for B. pumilus was plotted. The mean freezing rate was 8.6°C/min. The pre-freeze spread plate count of B. pumilus was obtained. The mean freezing rate was 8.6°C/min. The pre-freeze spread plate count of B. pumilus was 5.3 $\pm$ 0.27 x $10^8$cfu/ml and the post-freeze count was 3.4 $\pm$ 0.17 x $10^8$cfu/ml giving a survival of 65% of the organism. No further deterioration in viable counts was detected within a year of freezing. The thawing rate was 78°C min when the ampouls were thawed as described above.

Microcalorimetric Analysis of Growth Curves

P-t curves of B. pumilus at 30$\mu$V full scale deflection (471.15$\mu$W) show a logarithmic increase in power. The p-t curves for incubations in presence of: 0.05% glucose showed a peak at approx 13h, 33% of total deflection; 0.1% glucose showed a peak at approx. 16h, 39% of total deflection; and 0.2% glucose showed a peak approx 17h, 65% of total deflection. After 10h growth the power output was: 17% for 0.05% glucose, 22% for 0.2% glucose and 18% for 0.1% glucose. It was decided to use 0.1% glucose for microcalorimetrically monitored growth experiments, because use of 0.2% glucose could result in the bacterium clumping in the calorimetric chamber or in the Teflon tubes leading to and from the calorimeter. In 0.05% glucose B. pumilus grew slightly slower and to a smaller percentage total deflection than in the 0.1 % glucose incubations. The p-t curve of 0.1 % glucose on 10$\mu$V (157.05$\mu$W full scale deflection) was obtained.

Analysis of p-t curves showed that the decrease in power output at 16h 20min was due to exhaustion of glucose in the medium. The initial bacterial count was 5.1 x $10^6$cfu/ml and at the peak 9.3 $\pm$ 0.47 x $10^8$cfu/ml (1 estimation) and at 17h 30min 1.8 $\pm$ 0.09 x $10^9$cfu/ml (1 estimation). The pH decreased with utilisation of glucose to a final pH of 7.3 after 17h 30min. No spores were observed during this period of growth (i.e. up to 17h).

The p-t curves for 0.1 % glucose incubation were reproducible at 7h to a degree of 97.5%. Standard deviation was $\pm$ 2.5% (10 determinations). The average power-output at 7h was 34.1% of full scale deflection at 10$\mu$V.

Antibiotic Work

All three antibiotics that were analysed were stored at -20°C. Solutions of these were also kept at -20°C and no noticeable deterioration in activity was noted over 1 year by alteration of their activity in the microcalorimetric assays. Polymyxin B sulphate was stored in solution at pH 7.0 (phosphate buffered). Neomycin sulphate was stored in solution pH 7.9 (phosphate buffered) znbacitracin was stored and made up as follows:

   100mg znbacitracin was accurately weighed and suspended in 5ml H2O (deionised). 0.5ml 3M HCl was added and after the antibiotic had been dissolved, the solution was made up to 11 with $H_2O$ (deionised). This solution was frozen down and used as stock solution (100 μg/ml). Stock solutions for polymyxin and neomycin contained 100 μg/ml. the activities of the three antibiotics were as follows:
Neomycin sulphate: 699iU/ml
Polymyxin B sulphate: 846.4iU/ml
Znbacitracin: 63.27iU/ml

MIC Determination

MIC and MBC's were determined as follows:

Two-fold serial dilutions of antibiotic in their respective media were incubated for 16-24h (depending on the bacterial strain) and assayed for turbidity.

A tube with a turbid suspension was not inhibited and a clear suspension with the lowest concentration of antibiotic showing no growth was designated MIC. MBC's were checked as follows: 0.01ml of each suspension from tubes showing no growth were added to agar plates and incubated for 48H; the lowest concentration of antibiotic from which no colonies formed was designated MBC.

Electron Microscopy

a) Freeze Fracture:

Cells were fixed in 5% glutaraldehyde w/w, washed twice in 0.17 M sodium cocadylate buffer pH 7.3 and suspended in 30% w/v glycerol. They were then frozen in "slushed" nitrogen at -20°C, freeze factured in a Polaron E7500 freeze fracture unit at -115°C, shadowed with platinum/carbon at approximately 45 degree angle and then replicated with carbon. Replicas were then floated in water and cleared with sodium hypo chlorite (12% w/v) for approximately 2h, then washed in water and picked up on 400 mesh uncoated grids. These were examined and photographed in a Philips EM301G transmission electron microscope (100kV).

b) Sectioning

Cells were fixed in 5% w/v glutaraldehyde washed twice in 0.1M sodium cocadylate buffer pH 7.3 and embedded in 2% w/v agar. These were then fixed in 1% w/v osmium tetroxide buffer for 2h, washed in water, then in 50% v/v ethanol/water for 30 min, 70% v/v ethanol/water for 20 min, 95% v/v ethanol/water for 20 min, 2x20 min in 100% ethanol, then overnight 1:1 acetone/spurr resin by polymerisation at 70°C for 18h.
Sections were cut on Reichart OMV4 ultramicrotome using glass knives and then picked up on uncoated 400 mesh grids. They were stained for 20 min by saturated uranylacetate in 70% w/v ethanol and for 5 min by 0.4% w/v lead citrate/water.
The sections were examined in the Pilips EM30/G transmission electron microscope (100kV).

Uptake experiments for Znbacitracin

The adsorption of Znbacitracin onto M. flavus cell walls was followed using atomic absorption spectroscopy. The uptake was investigated under various conditions. Variations in temperature, pH and concentration was observed. The amount of Zn in Zn bacitracin was assayed using atomic absorption spectroscopy. (Perkin Elmer SB900).

Materials and Methods

Znbacitracin: was made up as described previously to a concentration of 100 μg/ml and then diluted with tris buffer to 5 μg/ml - 20 μg/ml.
Zn standard: A 500 μg/ml stock solution of Zn (11) was made up weekly and kept in a plastic bottle to avoid exchange of Zn with Zn in glass bottles.

A Typical Incubation

A 250 ml conical flask, containing a stirrer was incubated at 30°C or 10°C. Tris buffer at appropriate pH and Znbacitracin were incubated for 10 min to reach the assay temperature. An ampoule was thawed and 1 ml was resuspended in 100 ml 0.9% w/w saline. 1 ml of this solution was added to 99 ml of the Znbacitracin/Tris solution at t=0. At regular intervals samples of 10 ml were taken, filter on a 23 mm Millipore filter and the filtrate was assayed for Zinc on the atomic absorbance spectrophotometer.

MICROCALORIMETRIC ANALYSIS OF NEOMYCIN SULPHATE USING BACILLUS PUMILUS AS TEST ORGANISM

Neomycin sulphate is a broad spectrum antibiotic and is active against Gram positive and Gram negative organisms.

Antibiotic Incubation

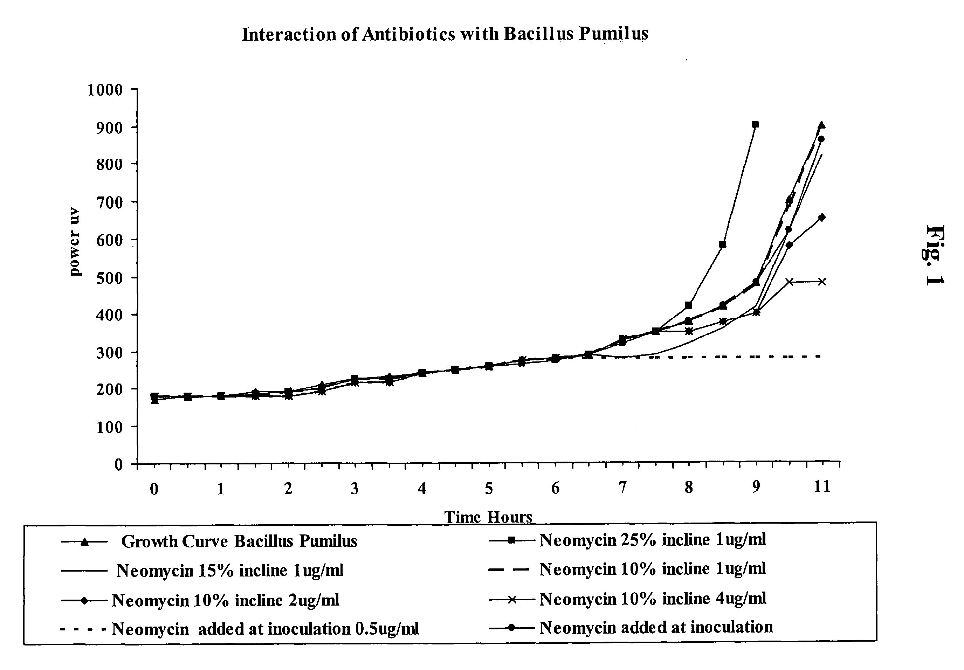

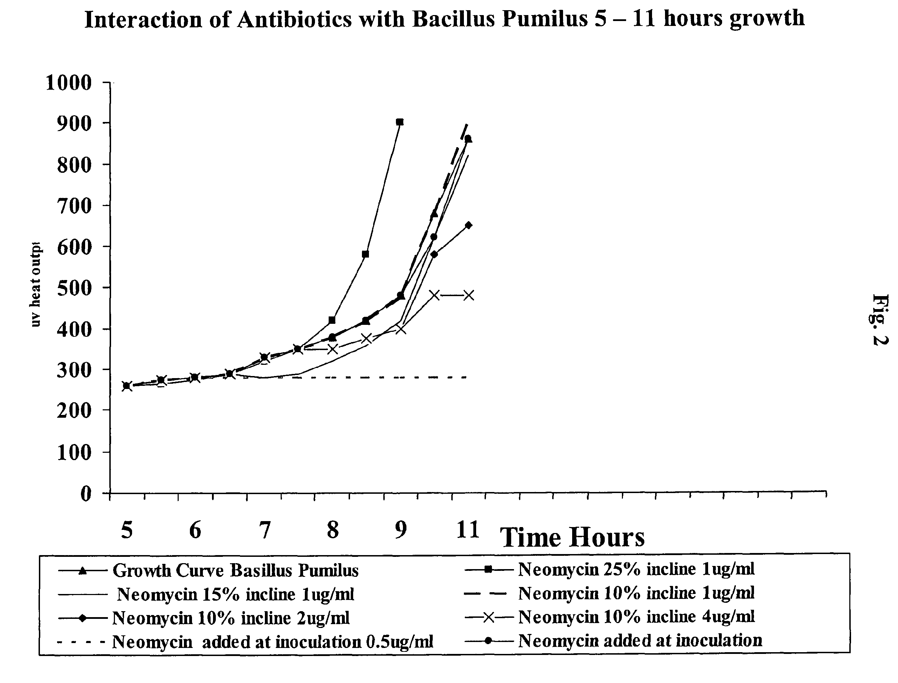

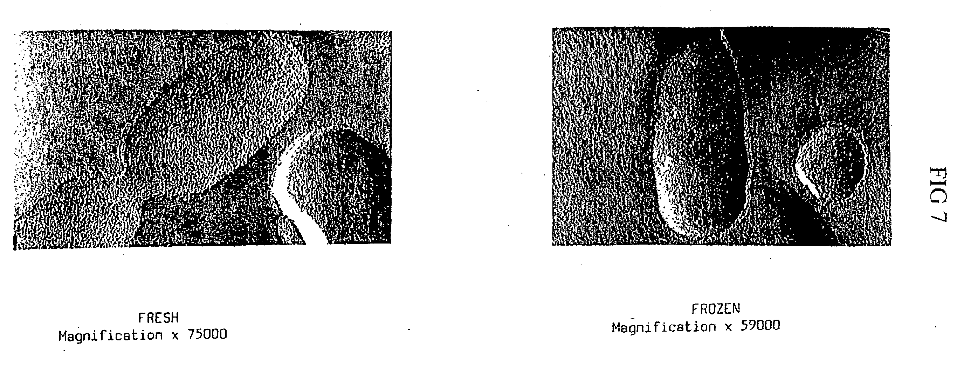

The MIC for neomycin sulphate against B. pumilus was found to be 0.1 $\mu$g/ml with an inoculum of $1\times10^5$ cfu/ml and the effect was bacteriostatic. However when the concentration of organisms was 5.1 x $10^6$ cfu/ml which was the inoculum used in the microcalorimetric incubations the MIC was increased to 2 $\mu$g/ml; the effect was also bacteriostatic.
When added after a 25% increase power output 0.5 x MIC (1$\mu$g/ml) neomycin gave rise to a massive power output 2-4h after addition. The effect of neomycin when added after an increase in power output of 15% of full scale deflection was only marginally larger than that observed for the normal growth curve.
When 0.5 and 1 x MIC (1-2 $\mu$g/ml) neomycin was added after an increase of 10% of full scale deflection the same effect was observed (Figs. 1 and 2). When higher concentrations of antibiotic were added (Figs. 1 and 2) a decrease in power output was observed. These observations indicate a mechanism of action for bacterial resistance to antibiotics, i.e. that when an antibiotic is used at suboptimal conditions (e.g at a lower concentration than the MIC) o, RNA synthesis is initialised that will within a short period of time provide proteins used as nutrients for the bacteria and thus the bacteria may grow at a rate which is greater than in the absence of antibiotic, and thus give rise to the notion that resistance is observed. This is supported by observations made during other uses of these antibiotics and is consistent with their growth promoting effects on poultry, dairy herds and pigs. More seriously when overtreating patients with antibiotics that have this effect eukaryotic protein synthesis may be initialised and act as a substrate for eukaryotic cell growth ie cancer or m RNA may be produced leading to expression of previously latent DNA such as observed in shingles infections after treatment with flagyl metronidazole an antibiotic commonly used in dentistry. (please see the section on neomycin treated E.coli where bacterial respiration is increased immediately on treatment with antibiotic indicating increased RNA synthesis. No difference between fresh and frozen bacteria could be observed by EM fig 7 Given the above it is possible to assess new agents on model systems such as this and to determine whether they have an effect on protein synthesis, respiration or growth of cells and whether any detrimental effects can be observed that could harm patients. Efficacy and safety can be demonstrated and used as a basis for new drug applications to regulatory authorities.
The results are summarised in table 1:

| Bacterial Concentration Bacillus Pumulus | Antibiotic Concentration | Observed effect on p-t curves | Comments |
|---|---|---|---|
| As % of p-t curve | Ug/ml MIC 1ug/ml | Related to standard growth curve (n=10) CV = 2.5% | |
| 25 | 1 | 5X signal at 11 h | Growth promoting effect **potential stimulation of RNA synthesis** |
| 15 | 1 | 1.2 X signal at 11 h | **Growth promoting effect potential stimulation of RNA synthesis** |
| 10 | 1 | 1.2 X signal at 11 h | **Growth promoting effect potential stimulation of RNA synthesis** |
| 10 | 2 | 1.2 Xsignal at 11 h | **Growth promoting effect potential stimulation of RNA synthesis** |
| 10 | > 2 | 0.75 or less at 11 h | Inhibition of RNA synthesis **Possible to achieve linear dose-response at 7 hours after addition of antibiotic to the bacterial culture, after 11 hours heat-out put levels off b ut do not return to baseline indicating a bacteriostatic effect and maximum inhibition of RNA synthesis** |
| 0 | 0.5 | O.75 of standard curve or less | **Inhibition of RNA synthesis** |
| 0 | 0.25 | 1X standard curve | No effect |

See figures 1 and 2

Assay of Znbacitracin against Microccous Flavus

It was found that addition of the antibiotic half way up the peak maximum would be convenient as the cells were growing exponentially at this point and the increase in power output was due to growth rather than baseline variation. This point occurred at an increase in deflection of 29.5% from the baseline value. See table 2:

| Bacterial Concentration Microccocus Flavus | Antibiotic Concentration | Observed effect on p-t curves | Comments |
|---|---|---|---|
| As % of p-t curve | Ug/ml MIC & MBC 0.2 ug/ml | Peak at 54% deflection N=10, CV= 1.5% | |
| 50 | 0.1-1 | Linear log dose response curve 2 hours after addition of antibiotic CV 1.8% | Peak of growth curve occurs 60 min later than standard curve |
| 50 | 1-4 | Linear log dose response curve 20 min after addition of antibiotic cv = 1.4% n= 10 | 4 ug/ml largest dose that could be distinguished any higher dose s gave identical dose response curves |
| 0 | 0.2 - 0.3 | 1.4 standard curve CV =2.3% , n=10 | Initial growth promoting effect - delayed peak after 9 hours |
| 0 | 0.6-1 | Total inhibition of growth | Bacteriocidal effect confirmed all bacteria are dead |
| 50 | 0.02 | Standard growth curve | **No inhibition observed** |
| Figure 3 | | | |

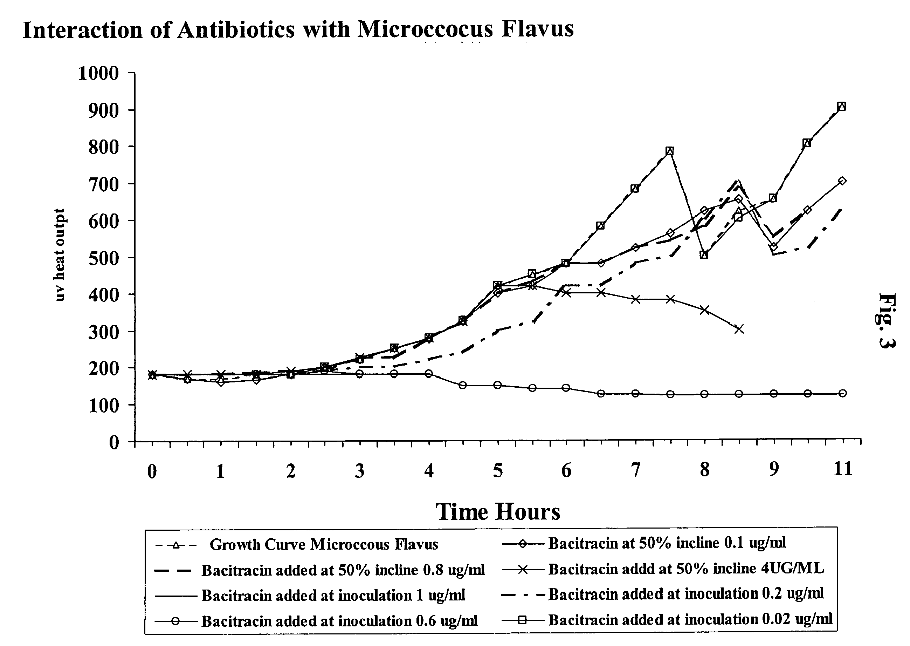

When adding Znbacitracin over the concentration-range (5 x MIC - 20 x MIC) the p-t curves (Fig. 3) showed the same general appearance: they were held at a plateau for a varying amount of time and then decreased towards a baseline value. A very rapid response of M. flavus to ZNB was observed making it possible to develop an assay only 20min after addition of the antibiotic. This was indicative of a possible effect on the bacterial membrane of the bacteria since peptidoglycan synthesis takes place between the cell membrane and the cell wall of the bacteria and inhibition of growth would not give much a rapid response.

When using concentrations of antibiotic from 0.02-0.8 $\mu$g/ml (0.1 x MIC-4 x MIC) the general trend was: a power plateau immediately after addition of the antibiotic for varying periods of time and then an increase in power followed by a further small peak which was observed at varying times - but in all cases later than for the control curve.

At concentrations lower than 0.02 $\mu$g/ml no variation in p-t curves could be observed and at concentrations higher than 4 $\mu$g/ml no further response could be recorded. The initial growth promoting effect at 0.2 -0.3 ug/ml of Bacitrazin when added at inoculum is consistant with observations of resistance patterns of penicillins also cell wall inhibiting agents and could be due to an effect on RNA synthesis similar to neomycin. Given the above it is possible to assess new agents on model systems such as this and to determine whether they have an effect on protein synthesis, respiration or growth of cells and whether any detrimental effects can be observed that could harm patients. Efficacy and safety can be demonstrated and used as a basis for new drug applications to regulatory authorities.

## EFFECT OF BACITRACIN ON THE CELL SURFACE OF MICROCOCCUS FLAVUS

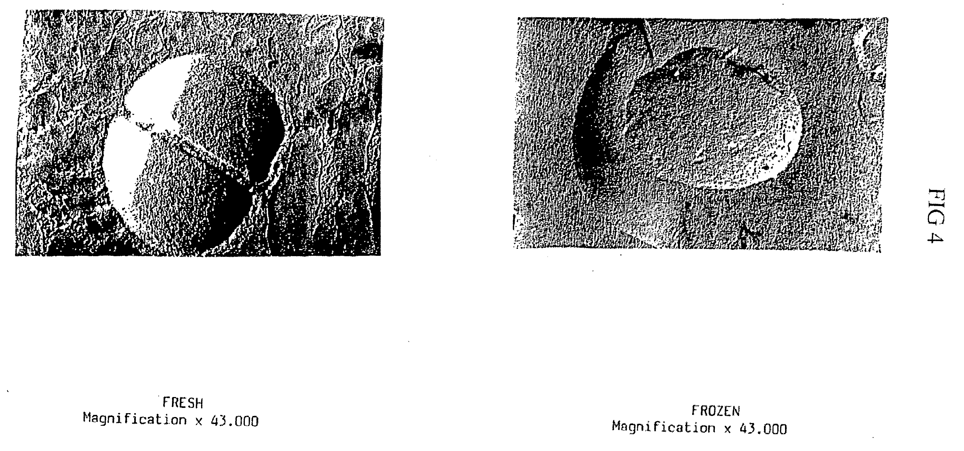

No difference could be observed between electron micrographs of M. flavus taken from frozen and fresh samples. (Fig. 4). This suggests that no damage to the bacterial cells occurred during the freezing process.

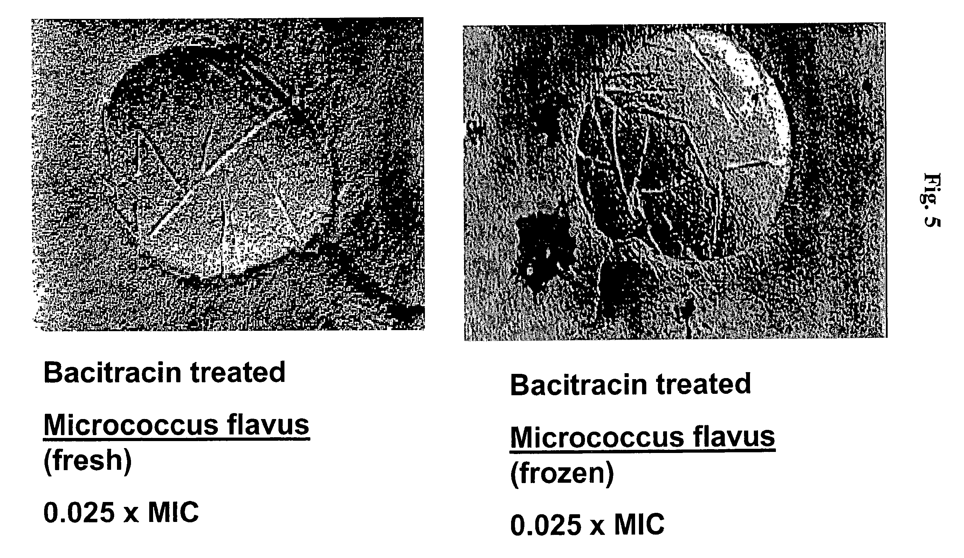

Fig. 5 shows EM's of samples treated with Znbacitracin in the same manner as in the antibiotic assay. Protrusions on the cell wall can be observed even at concentrations of 0.05 $\mu$g/ml (0.25 x MIC). No differences could be observed between fresh and frozen cells treated with antibiotic, which is as expected since no observable damage had occurred to the bacterial cells during freezing/thawing.

The sensitivity of this assay is very high as levels of 0.02 $\mu$g/ml can be measured. This is because much better control of the different parameters is achieved than would be possible for a plate assay. This is interesting as no ordinary microbiological assay can compete in sensitivity and this is achieved under conditions which more accurately represent *in vivo* conditions than any other assay available.

The reproducibilities of these two assays $\pm$1.4% and $\pm$2.3% are excellent. They fall in the category of reproducibilities of quantitative chemical analysis which is extremely good for a microbiological bioassay. It is remarkable that these reproducibilities can be achieved using only a semi-defined medium and therefore not achieving as great a degree of control over parameters as when using a defined medium

MICROCALORIMETRIC BIOASSAY OF POLYMYXIN B SULPHATE USING BORDETELLA BRONCHISEPTICA AS TEST ORGANISM

Polymyxin B sulphate is a membrane active antibiotic mainly active against Gram negative organisms. Bord. bronch, is the reference test organism for this antibiotic. The defined medium finally arrived at for this organism has been described previously. Ordinarily Bord. bronch, is grown in complex medium containing proteose and yeast extract.

Microcalorimetric Analysis of P-T Curves

After successful freezing of Bord. bronch p-t curves for growth in different concentrations of glutamic acid from 0.1%-0.5% were obtained. It was concluded that a medium containing 0.2% glutamic acid would be sufficient for a good deflection in the microcalorimeter minimising the possibility of the bacteria clogging in the microcalorimetric cell or in the teflon tubes leading to and from the microcalorimetric cell. A pH of 7 was used in all the following experiments as this is the optimum pH for polymyxin - lipid binding.

It was of vital importance to maintain a constant oxygen tension; this was achieved by using saturation concentrations of oxygen. It was decided to use the height of the small peak as full scale deflection for the antibiotic assay as the increase in power was exponential up to this point and the peak occurred at a convenient time. This choice of assay point was empirical. The inoculum was $1.1 \pm 0.06$ x $10^8$ cfu/ml for all the microclalorimetric incubations.

| Bacterial Concentration Bordetella Bronchiceptica | Antibiotic Concentration | Observed effect on p-t curves | Comments |
|---|---|---|---|
| As % of p-t curve | Ug/ml MIC & MBC 2 ug/ml | Peak at 39% deflection N=10,CV=2.6% | |
| 18 | 0.35 -2 ug/ml | Linear log dose response 10 min after addition of antibiotic CV 2.5% | All curves return to baseline confirmed bacteriocidal action |
| 18 | 0.07 ug/ml | No effect on growth curve | |
| Fig6 | | | |

Analysis of the p-t curves of 0.2% glutamic acid revealed that the small peak occurred between 5h 30min-6h 30min. The average peak height was 39% of full scale deflection (at $10\mu V = 157.05\mu W$). The standard deviation in peak height was $\pm 2.6\%$ over ten measurements.

Analysis of control incubations without antibiotic and with 0.2% glutamic acid showed that after a lag of 1h 20min OD and heat output increased exponentially up to the first peak (39% deflection, 5½-6½h). The viable count during this period increased from $1.1 \pm 0.06$ x $10^8$ to $3.5 \pm 0.18$ x $10^8$ cfu/ml, pH did not change. Glutamic acid utilisation during this period was not detectable.

Antibiotic Incubations

It was found that one doubling of cells ($1.9 \pm 0.1$ x $10^8$ cfu/ml) occurred at an increase in deflection of 17.5% (2 determinations). This was a suitable point for addition of antibiotic as the cells at this point were growing exponentially and the increase in power was great enough to ascertain that any increase in deflection was due to growth rather than a baseline variation.

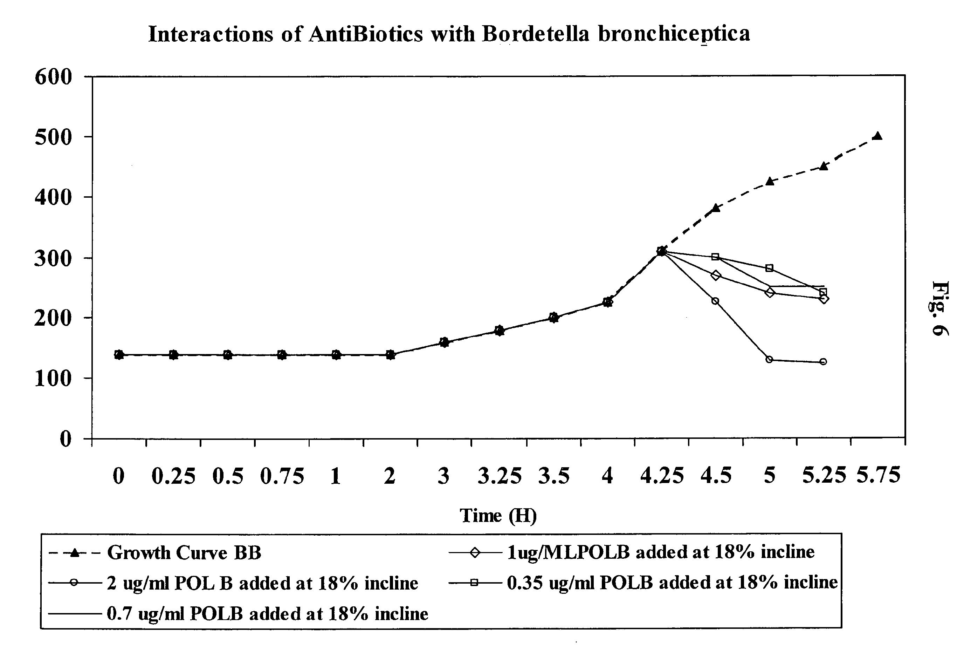

A concentration of 0.5 x MIC (MIC and MBC: $2\mu g/ml$) was added and a p-t curve with a sharp decline in heat output within 10 min of addition resulted. A range of different concentrations of polymyxin B sulphate was assayed in the microcalorimeter Fig. 6. It can be seen that the rate of change in decline of the p-t curve increases with increase in concentration of antibiotic. At concentration larger than 3.5 x MIC the declines were identical. At a concentration of 0.033 x MIC (0.07 $\mu g/ml$) no response to the antibiotic was observed. At small concentrations of antibiotic the decline became very shallow.

The sensitivity range for the assay of polymyxin using the slope as response was $0.35\mu g/ml$ (0.175 MIC) - 2µg/ml (1 x MIC). Although a response was seen at lower concentrations of antibiotic the decline in power was small and difficulties arose in distinguishing slopes at these concentrations. The standard deviation for this assay was ± 2.9% (10 measurements). It can be concluded that the uptake of polymyxin was very quick (a response could usually be observed within 5 min of addition of the antibiotic) and that the effect was irreversible for most concentrations of antibiotic as the decline in power decreased to a baseline value. This was in accordance with the mode of action of the antibiotic being bactericidal at MIC values.

Given the above it is possible to assess new agents on model systems such as this and to determine whether they have an effect on protein synthesis, respiration or growth of cells and whether any detrimental effects can be observed that could harm patients. Efficacy and safety can be demonstrated and used as a basis for new drug applications to regulatory authorities.

### Effect of POLB on the Cell Surface of Bordetella Bronchiseptica

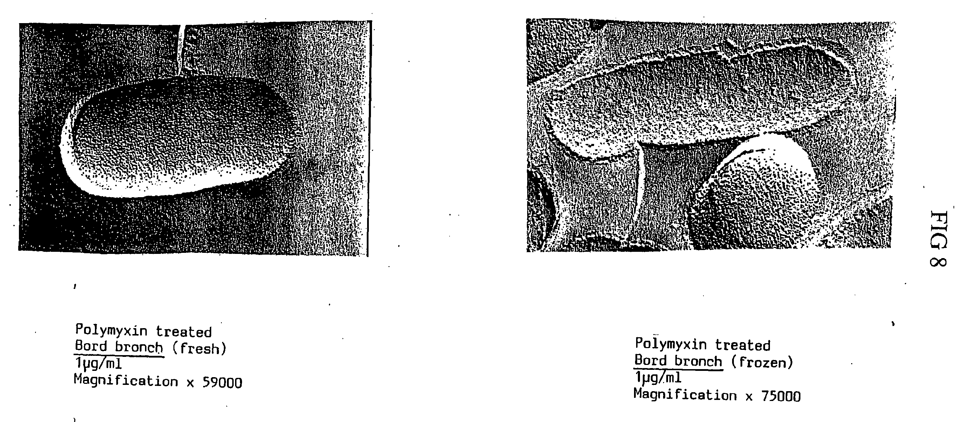

No difference could be observed between electron micrographs of <u>Bord. bronch</u>, taken from fresh and frozen samples (Fig. 7). This suggests that no damage to the bacterial cells occurred the freezing process. After incubating fresh and frozen bacteria with the antibiotic, in the same manner as in the antibiotic assay at concentrations of 1µg/ml (0.5 x MIC) no differences were observed between the samples (Fig. 8).

### Discussion

The accuracy of the flow microcalorimetric assay is high. The parameter most likely to cause any variation in accuracy would be a variation in antibiotic standards. The sensitivity of this assay is also very high. This can be seen from the results of the EM's of the bacteria where no visible damage to the bacteria can be detected within the sensitivity ranges of the assay implying that very small metabolic changes can be detected in the calorimeter. This is important as conventional microbiological assays can not compete with the sensitivity which is obtained at concentrations close to those found "*in vivo*".

Flow - microcalorimetry is a very simple method to use for routine assay of antibiotics, when all the conditions discussed previously are observed. For this assay the standard deviation was ± 2.9% which is a great improvement in reproducibilities from plate assays. (See table ref below).

| Criteria | Calorimetry | Plate Method |
|---|---|---|
| Inter Assay Reproducibiity % | 3 | 5-10 |
| Sensitivity u/ml | 0.1 | 20 |
| Range u/ml | 0.1-100 | 20-100 |
| Time per assay (h) | 1 | 16 |
| Capacity for automation | High | low |
| Convenience and simplicity | High | high |

### ASSAY OF MIXTURE OF ANTIBIOTICS

Even in the earliest days of the antibiotic era, despite dramatic cures of what had previously been fatal infections, it was apparent that in certain situations the use of a single drug led to unacceptable failure rates.

The exact role of antibiotic combinations in current therapy of clinical infections often remains controversial or unclear, in part because few tightly controlled studies have been carried out in this area.

Compatibility of different antiinfectives or indeed non compatibility involves several mechanisms, including sequential blockage in a given metabolic pathway e.g. trimethoprim and sulfonamides or changes in permeability to the bacterial surface by one drug e.g. penicillin and aminoglycosides or inhibition of inactivating enzymes e.g. penicillin and cloxacillin.

Results have varied when combinations of aminoglycosides and antpseudomonas penicillins have been used against <u>Ps. aeruginosa</u> and when combinations of cephalosporins and aminoglycosides have been used against Klebsiella species. However it is clearly unusual for non compatibility to be suggested by one technique when another method shows compatibility of an antibiotic combination against the same organism.

In the interpretation of the results of all tests of *in vitro* antimicrobial activity, attention must be paid to the overwhelming importance of host factors in the outcome of treatment of any infectious process. *In vitro* tests do not usually take into account the role of phagocytic cells, humoral antimicrobial substances, pharmacokinetic factors and pathopysiological conditions at the site of infections. There are techniques to establish bactericidal effects in blood but these have serious microbiological limitations as regards performance and interpretation of results. The results depend on how the test was done e.g. on the salt concentration and other environmental parameters. However some correlation could be achieved between the test and the clinical outcome.

No clinical studies have been carried out in which an antibiotic combination consistently compatible with another against an infecting organism was compared to a single agent with *in vivo* activity against the infecting pathogen.

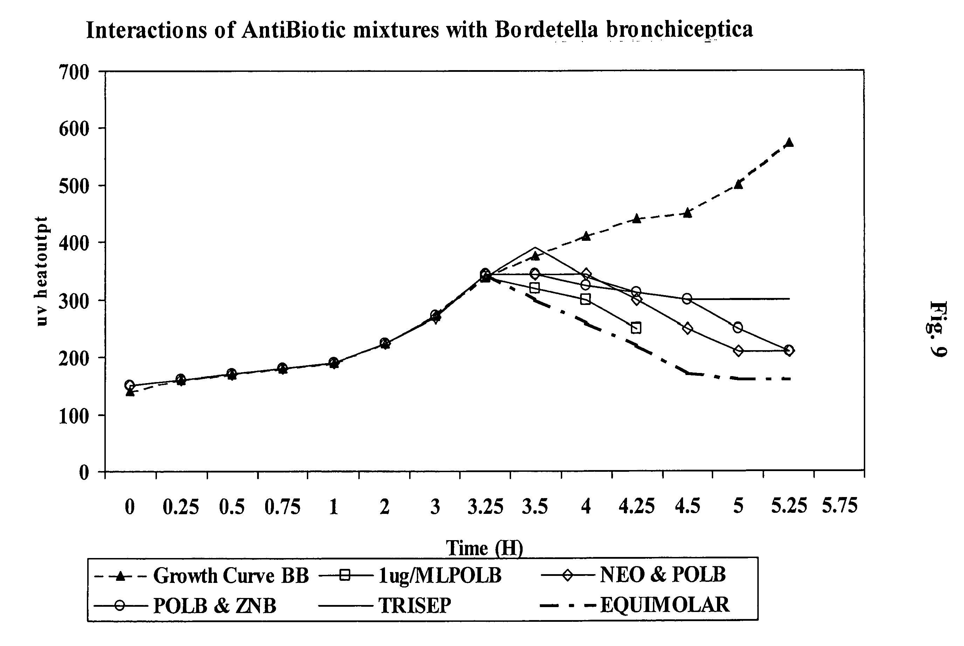

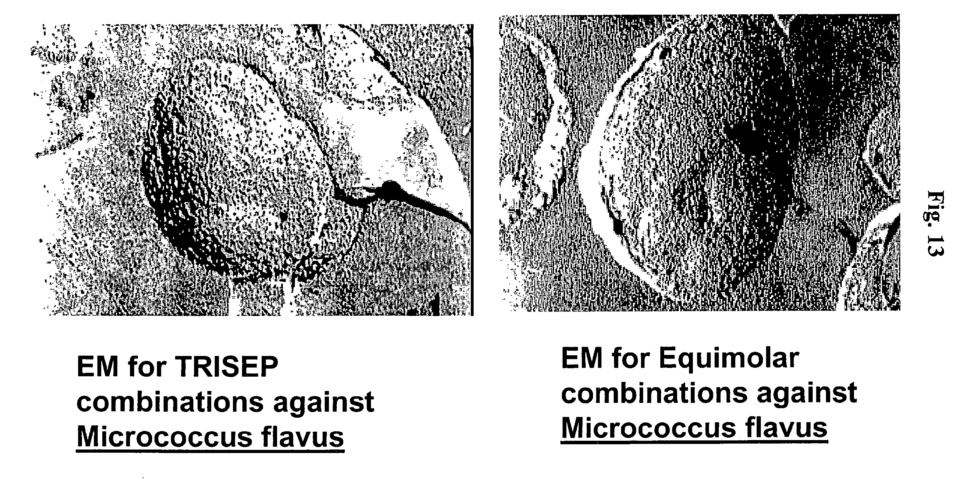

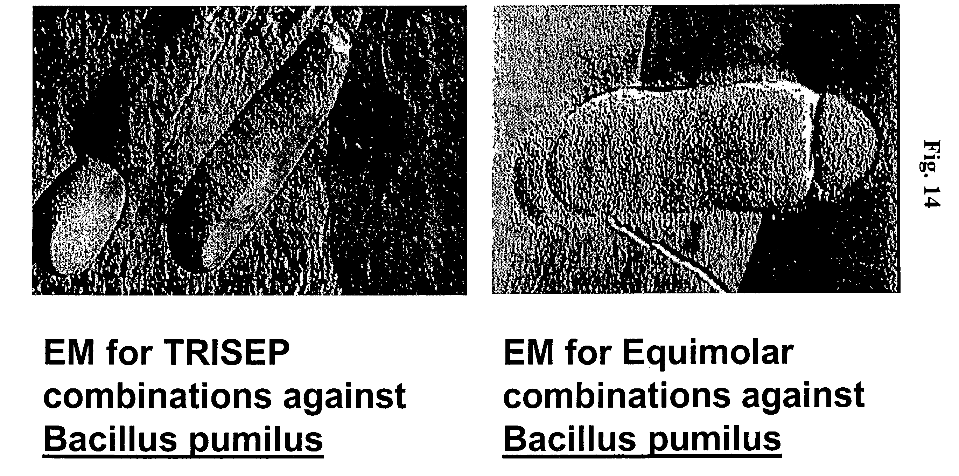

Biological compatibility and non compatibility of antibiotics can be demonstrated by micro calorimetry (see Figures 9 to 18). The antibiotics assayed in combination in this study were POLB, NEO and ZNB. These antibiotics were available as an antibiotic spray "TRISEP" from ICI and contained, on average, NEO 500.00 u, POLB: 150.000 u and ZNB 40000 u per can. Converted to mg the content was NEO 7.5 x $10^2$ mg, POLB 1.7 x $10^2$ mg and ZNB 6.32 x $10^2$ mg. When assaying antibiotics in combination one antibiotic is usually added at some multiple of its MIC, whereas the amount of the second or third antibiotic added is often empirically gauged. A simpler and more regular picture of the interaction of mixtures of antibiotics would be achieved by using equimolar concentrations or concentrations taking into account the mode of action of the antibiotics. In this study antibiotic mixtures have been assayed in the proportions found in the Trisep mixture and in equimolar concentrations. The relative molecular masses (RMM) for the three antibiotics were as follows: ZNB: RMM 1477, POLB: RMM 1300 and NEO RMM 700.

MIXTURES OF ANTIBIOTICS ASSAYED AGAINST BORDETELLA BRONCHISEPTICA (see Fig. 9)

| Bacterial Concentration Bordetella Bronchiceptica | Antibiotic Concentration | Observed effect on p-t curves | Comments |
|---|---|---|---|
| As % of p-t curve | Ug/ml MIC NEO 0.25 ug/ml ZNB 100ug/ml POL 2ug/ml | | |
| 50 | 0.25ug/ml Neo | No effect on p-t curve | |
| 50 | 100ug/ml ZNB | Slightly delayed peak | |
| 50 | Pol 1 ug/ml, Neo 4 ug/ml (Trisep conc) | Sharp decline in curve 10 min after addition of antibiotics response polB 0.7 ug/ml | Non compatibility of antibiotics demonstrated |
| 50 | POL 1 ug/ml ZNB 3.57ug/ml | 0.39 ug/ml POLB | Non compatibility of antibiotics demonstrated |
| 50 | All three antibiotics trisep combinations | 0.47 ug/ml Pol B | **2 hours after addition of antibiotic pt-power out put declined 10%, No return to baseline** |
| 50 | All three antibiotics equimolar amounts | 0.39 ug/ml PolB | **2 hours after addition of antibiotic pt-power out put declined 20% No return to baseline** |
| Fig 9 | | | |

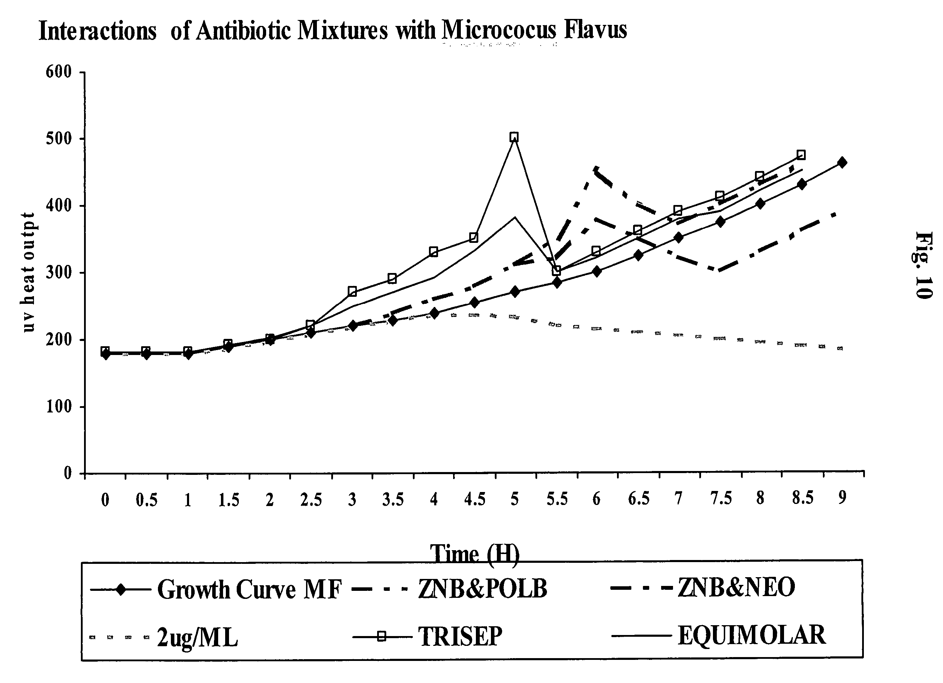

MIXTURES OF ANTIBIOTICS ASSAYED AGAINST MICROCOCCUS FLAVUS (see Fig. 10)

| Bacterial Concentration Micrococcus flavus | Antibiotic Concentration | Observed effect on p-t curves | Comments |
|---|---|---|---|
| As % of p-t curve | Ug/ml<br>MIC NEO 4 ug/ml<br>ZNB 0.2ug/ml<br>POL 2ug/ml | | |
| 0? | 4 ug/ml Neo | No effect on p-t curve | |
| 0? | 2ug/ml POLB | Slightly delayed peak | |
| 50 | 0.2 ug/ml ZNB 056 ug/ml POLB (trisep) | Delay in peak ZNB conc 0.11 ug/ml | Non compatibility of antibiotics demonstrated |
| 50 | NEO 0.226 ug/ml ZNB 0.2ug/ml (trisep) | 0.1 ug/ml ZNB | Non compatibility of antibiotics demonstrated |
| 50 | All three antibiotics trisep combinations | 1.3 times standard cuve | **Growth promoting effect** |
| 50 | All three antibiotics equimolar amounts | 0.1 ug/ml ZNB | **Small delay in peak compared to standard curve** |
| Fig 10 | | | |

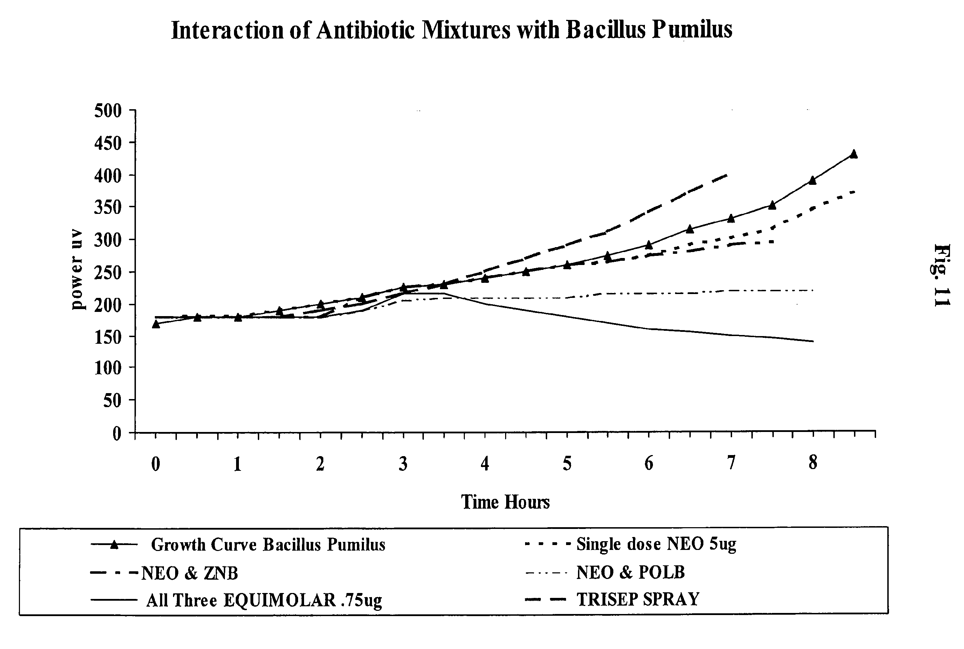

MIXTURES OF ANTIBIOTICS ASSAYED AGAINST BACILLUS PUMILUS (see Fig. 11)

| Bacterial Concentration Bacillus Pumilus | Antibiotic Concentration | Observed effect on p-t curves | Comments |
|---|---|---|---|
| As % of p-t curve | Ug/ml<br>MIC NEO 1 ug/ml<br>ZNB 20 ug/ml<br>POL 20 ug/ml | | |
| 0? | ZNB 20 ug/ml | No effect on p-t curve | |
| 0? | 20ug/ml POLB | Within 9 hours growth was inhibited completely | |
| 0 | 0.5 $\mu$g/ml) NEO + 0.125 $\mu$g/ml (0.006 MIC) POLB (TRISEP) | Compatibility Effect 1 ug/ml neomycin | compatibility of antibiotics demonstrated |
| 0 | And 0.25 MIC (0.5 $\mu$g/ml) NEO + 0.44 $\mu$g/ml 0.022 MIC ZNB (TRISEP). (trisep) | Compatibility Effect 1 ug/ml neomycin | compatibility of antibiotics demonstrated |
| 0 | All three antibiotics trisep combinations | 1.0 times standard cuve | **No effect** |
| 0 | All three antibiotics equimolar amounts neomycin I ug/ml, POLB, ZNB | 1.7ug/ml Neomycin | **Compatibility demonstrated** |
| Fig 11 | | | |

DISCUSSION

Compatibility was clearly demonstrated for all antibiotic combinations against <u>B. pumilus</u> except the Trisep mixture. These studies were all carried out at pH 8.00 as this was the most effective pH for neomycin. It seems likely that when using the Trisep mixture POLB and ZNB interfered with each other whereas an equimolar concentration no such effect was seen. The results suggest a critical range of concentration within which compatibility is observed.

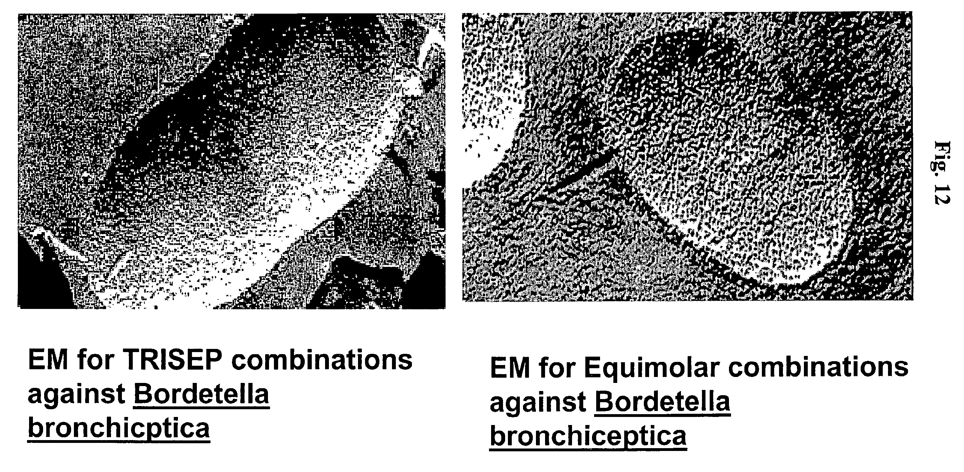

When assaying the three antibiotics against <u>Bord. Bronch</u> and <u>M. flavus</u> no such effect could be observed. In fact an indication of non compatibility could be observed for all these mixtures, and even a growth promoting effect at lower concentrations of antibiotics. It seems likely that the compatible action against Bacillus pumilus was due to POLB and ZNB facilitating the transport of neomycin into the cytoplasm by disrupting the bacterial membrane. All the above results have been verified by electron microscopy (see Figs. 12 to 14). It has been shown by EM that ZNB had some effect on <u>E. coli</u> as protrusions have been observed on the bacterial membrane after treatment with ZNB.

A possible assay of POLB and ZNB in the combinations used here could probably be achieved using <u>Bord. bronch</u> and <u>M. flavus</u> respectively as test organism. The assays would not be as sensitive as the assays for the individual antibiotics but a linear relationship between dose and response may be achievable. For the neomycin assay, using <u>B. pumilus</u> as test organism, a great variation in concentration of the antibiotics would have to be carried out to see at which concentrations, if any, an assay of all three antibiotics would be possible i.e. that an assay could be developed for neomycin with a constant response for ZNB and POLB.

Given the results described above it is possible to assess new agents on model systems and using the three test organisms described above gives an excellent basis for development of new antibiotics or other growth inhibiting agents and to determine whether they have an effect on protein synthesis, respiration or growth of cells and whether any detrimental effects can be observed that could harm patients. Efficacy and safety can be demonstrated and used as a basis for new drug applications to regulatory authorities.

RESPIRATION AND GROWTH OF E.COLI

<u>E.coli</u> was suspended in 10% DMSO prior to freezing and the viable counts were for batch 1: pre-freeze: 4.0 $\pm$ 0.6_ x $10^{10}$ cfu/ml and post -freeze 2.0 $\pm$ 0.44 x $10^{10}$ cfu/ml and for batch 2: pre-freeze: 3.45 $\pm$ 0.58 x $10^{10}$ cfu/ml and post-freeze 2.93 $\pm$ 0.43 x $10^{10}$ cfu/ml. The freezing rate for both batches was 20°C/min the survival rate was 50% for batch 1 and 85% for batch 2. These differences may have been due to batch 1 being kept for 24h at 4°C before being frozen.

The respiration experiments were carried out in glucose buffer at different pH. The respiration curves obtained from batch 1 (inoculum 2 x $10^{10}$ cfu/ml) and batch 2 Inoculum 3.2 x $10^{10}$ cfu/ml) were essentially similar and a mean was used as a reference). The respiration experiments were carried out at pH 7 for polymyxin and Znbacitracin and at pH 8 for neomycin. No differences in p-t control curves could be observed at pH 7 or pH 8.

The p-t curves rose and reached a plateau as has been observed for respiration of yeasts and some 20 min later fell to a lower plateau value. Prior incubation of the cells in the buffered glucose for periods up to 20 min before presentation to the calorimeter could reduce or entirely remove this effect.

INTERACTION OF POLYMYXIN B, NEOMYCIN AND ZNBACITRACIN WITH RESPIRING E. COLI

| Bacterial Concentration E. coli | Antibiotic Concentration | Observed effect on p-t curves | Comments |
|---|---|---|---|
| **As % of p-t curve** | **Ug/ml MIC&MBC NEO 5900 ug/ml ZNB > 1000 ug/ml POL 6.3ug/ml** | | |
| respiration | **POLB 6.3 ug/ml** | Reduced height of p-t curve initially and returned to baseline within I hour | **Bacteriocidal action demonstrated** |
| respiration | NEO 500 ug/ml | Increase in heat output Linear dose-response between 10-100ug/ml | **Consistent with increased rate of RNA synthesis** |
| respiration | ZNB 300 ug/ml - 1000 ug/ml | No response | Resistance demonstrated |

(continued)

| Bacterial Concentration E. coli | Antibiotic Concentration | Observed effect on p-t curves | Comments |
|---|---|---|---|
| respiration | : POLB: 20μg/ml NEO: 80.8μg/ml and ZNB 71.4μg/ml (TRISEP combination). | POLB - NEO longer plateau drop after 20 min to baseline POLB ZNB NEO ZNB NEO response curve | POLB/NEO interaction: delayed response compared to polymyxin alone but drop to baseline no increase in power NEO/ZNB no interference of ZNB with NEO action higher output but not as high as with NEO alone |
| respiration | All three antibiotics trisep combinations | Polymyxin curve with delayed return to baseline 20 min | **Effect not as good as polymxin alone or Neomycin alone** |
| respiration | All three antibiotics equimolar amounts | Polymyxin curve with delayed return to baseline 9 min | **Effect not as good as polymxin alone or Neomycin alone** |
| Fig 15,16 and 18 | | | |

### Interaction of Polymyxin B with E.coli

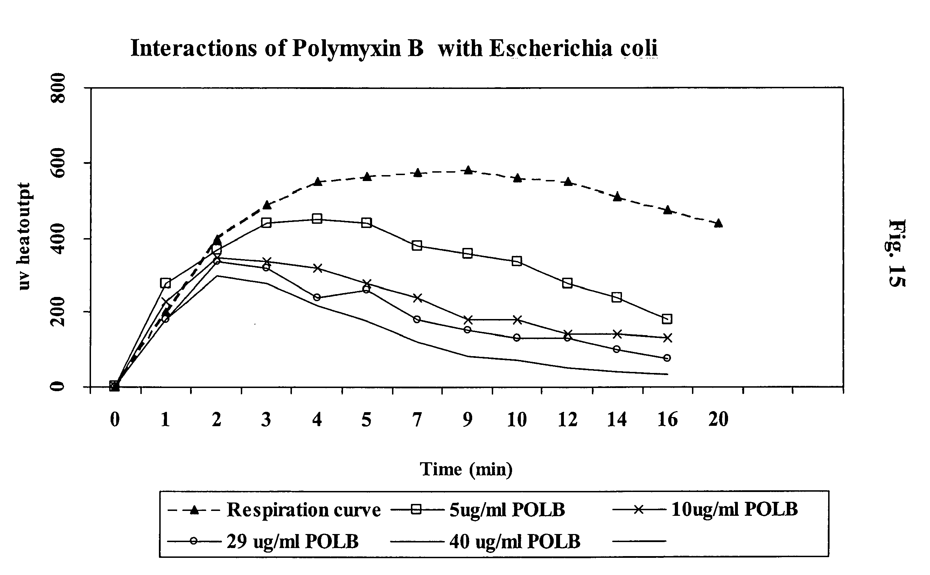

The MIC and MBC for E.coli was found to be 6.3μg/ml. Polymyxin B reduced the initial peak height and thereafter the p-t curve returned to baseline (Fig. 15). This effect was in accordance with the mode of action of polymyxin as the bacteriocidal action was very rapid. This was also observed when using Bord. Bronch as test organism.

### Interaction of Neomycin with E.coli

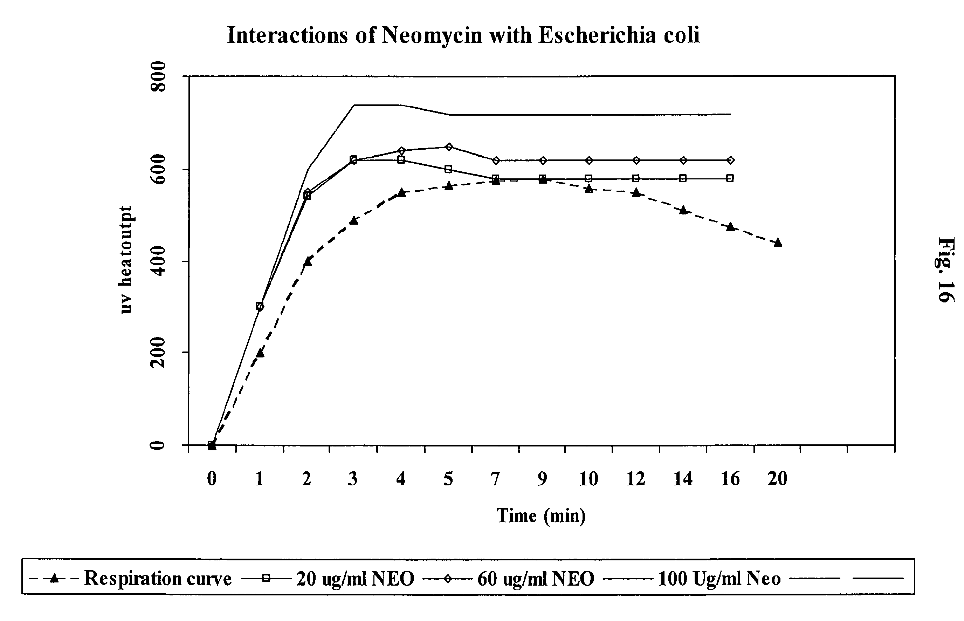

The effect of neomycin on E.coli is given in Fig. 16. In contrast to polymyxin, neomycin caused an increase in power which confirms that Neomycin fundamentally stimulates RNA synthesis and only inhibits it when present in very large amounts when added to respiring E.coli. (MIC: 500μg/ml). A linear relationship between dose and the reciprocal of increase in response from the control curve was found between 20-100μg/ml neomycin.

### Interaction of Znbacitracin with E.coli

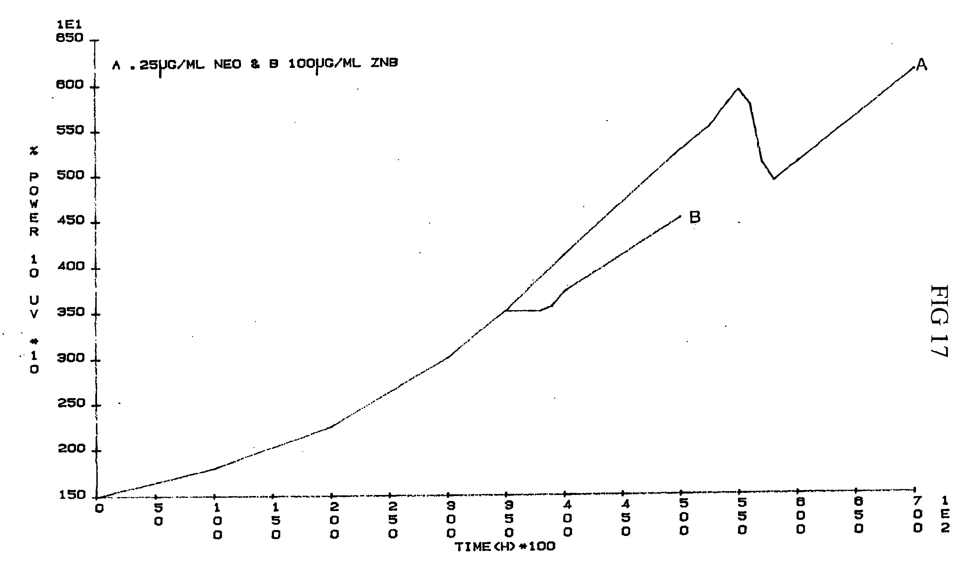

No response could be observed on addition of Znbacitracin to respiring E.coli. The highest concentration added was 300μg/ml (MIC> 1000μg/ml). ZNB has been reported to be inactive against E.coli. However electron micrographs have shown that ZNB had an observable effect on E.coli i.e. did actually cause protrusions to occur on the bacterial membrane. This response was not lethal for the bacterium but small changes in heat could possibly have been expected to occur. This has in fact been observed when ZNB was added to growing cultures of Bord. bronch. a small plateau was reached before growth reoccurred (see Fig. 17).

### Interaction of Mixtures of Antibiotics

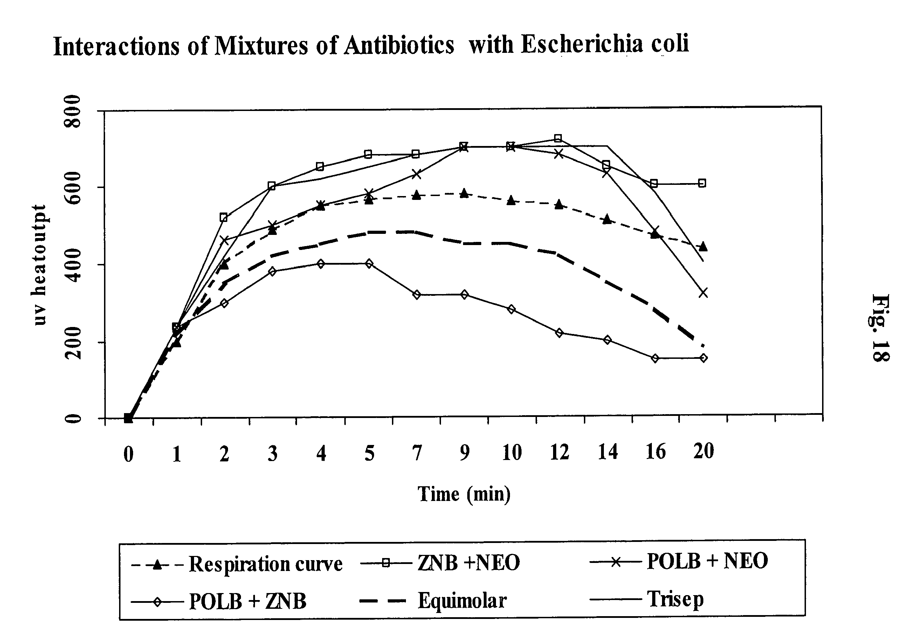

Fig. 18 shows the p-t curves for the effect of mixtures of antibiotics on E.coli. The concentrations were: POLB: 20μg/ml NEO: 80.8μg/ml and ZNB 71.4μg/ml (TRISEP combination). Fig. 18 shows the p-t curve for ZNB + NEO; when compared with the p-t curves for neomycin alone it can be seen that this p-t curve was essentially similar to that for neomycin alone (20μg/ml NEO). Fig. 18 shows the effect of POLB + NEO on E.coli. When comparing the effect of the mixture to that of POLB alone (Fig. 15) it can be seen that the effect of the two antibiotics was not comparable to the polymyxin interactions as the kinetics of the interactions seemed to have changed resulting in a plateau value for much longer periods than found for the polymyxin alone followed by a decline to a baseline value after 20 min. When compared to the neomycin interactions (Fig. 16) the p-t curve for NEO and POLB was very different.

Fig. 18 shows the effect of polymyxin and Znbacitracin in combination and when compared with that for polymyxin alone (Fig. 9) the p-t curve is seen to be similar in shape to that for polymyxin but shows a higher output. This response was equivalent to a response of 15μg/ml polymyxin and suggests non compatibility.

Fig. 18 shows the effects of all three antibiotics added to respiring E.coli (A) as the TRISEP mixture and (B) as the equimolar mixture. The TRISEP mixture shows a response similar to that of POLB + NEO apart from showing a decline to baseline which was at a slower rate whereas the equimolar mixture at (20μg/ml POLB) showed a polymyxin type curve with a plateau value very similar to that of the control curve, but then, after 9 min, a steady decline to baseline.

The heat effects observed with polymyxin B were those that would be expected of membrane active antibiotics; i.e. severe reduction in power output followed by a return to baseline signifying death or lysis of cells after a breakdown in membrane potential and leakage of cell constituents from the cytoplasm of the cell. The polymyxin type interactions showed kinetics which were essentially simply and which have been observed with antifungal antibiotics. Neomycin, in contrast, caused an increase in power output also observed in growth studies with Basillus Pumilus which would indicate an immediate effect on RNA synthesis and a very good reason not to use this antibiotic

That ZNB had no effect on power output was not surprising since the antibiotic has been reported to be active only against Gram positive organisms. The assays for POLB were not particularly sensitive (3μg/ml) but had the advantage of being rapid. They do compare favourably with the assay using Bord. Bronch. as test organism for this reason. Which assay to use would depend entirely on the sensitivity required. The reproducibilities for the E.coli assay were also smaller than those for the Bord. bronch. assay. The neomycin assay was much more sensitive when using B.pumilus as test organism. However the assay with E.coli was possible at concentrations much lower than the measured MIC (500μg/ml). It can be concluded that these two assays would be suitable for screening antibiotics where sensitivity is not essential, since they are very rapid (1h).

It can be concluded from Fig. 18 that all the antibiotics assayed together show (two by two) possible antagonistic effects. The effect observed with neomycin and polymyxin was an initial increase in peak deflection followed by a gradual decrease to baseline. This suggested that the initial effect on E.coli was due to neomycin followed by the effect of polymyxinFor POLB + NEO assayed against E.coli it can be observed that the rate of decline of power was identical to that noted for the antibiotic on its own suggesting that these interactions followed the same order of kinetics at this point. That polymyxin and bacitracin assayed together showed non compatibility was not surprising since a possible explanation could be that competition for binding sites on the bacterial membrane would occur. This effect was also in agreement with the effect observed for this mixture when assayed against Bord. bronch. and M. flavus. This possible competitive effect could result in the inhibition of binding of the most active agent. When all three antibiotics were added together either as the TRISEP combination or as the equimolar combination, p-t curves resulted that were different from those observed either for the antibiotics added singly or in binary combinations.

These results form an excellent basis for rapid compatibility testing of anti-infective agents both during development and also as part of clinical practise in hospitals, doctors offices, dental clinics, veterinary clinics and in development countries

An example for rapid efficacy and safety assessment of new compounds using respiration studies.

STRUCTURE-ACTIVITY RELATIONSHIPS OF NITROFURANS BY MICROCALORIMETRY

The antibiotic activity of chemically related compounds can be measured using flow-microcalorimetry. Addition of equimolar amounts of the compounds to be assayed to respiring cultures of E.coli would give an indication of the antibacterial activity of the compounds. The nitrofurans only differed in the length of their side chains (e.g. methyl, propyl etc.). The relative molecular masses (RMM) of the compounds assayed in this study were 1: 533.44 2:494.43 3: 524.52 and 4: 281.54.

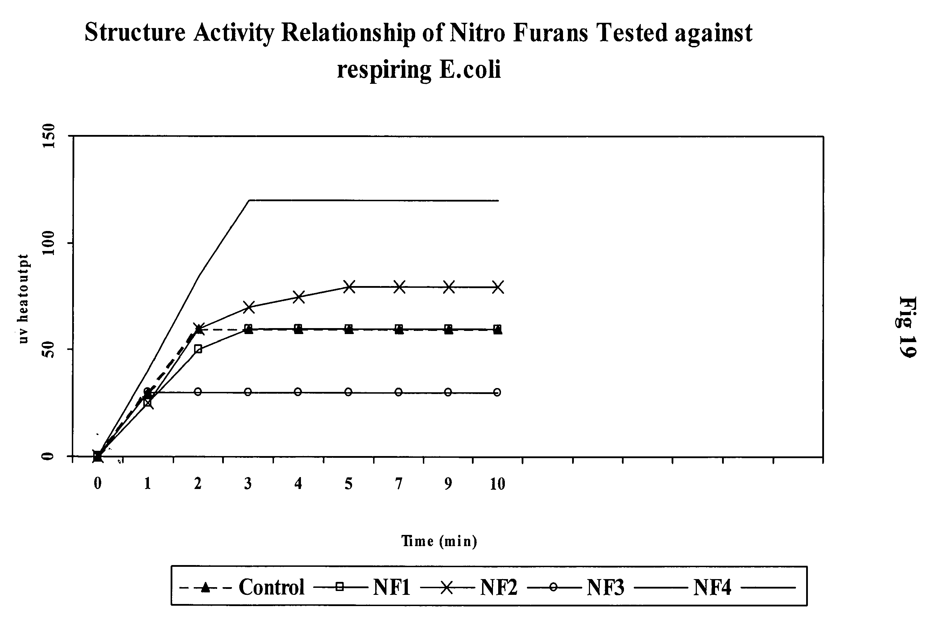

To assay the nitrofurans at a concentration where the maximum sensitivity to E.coli could be achieved MIC's were determined for each compound. They were as follows: 1: 10mM 2: 0.625mM 3: 0.157mM 3: MBC: 0.313mM 4: >10mM. It was decided to use 0.625mM as assay concentration as differences in response to control curves can often be achieved at concentrations lower than MIC. The respiratory medium was the one described previously at pH7. 1.94 $\pm$ 0.1 x $10^{10}$cfu/ml of E.coli treated as described previously was found to give a satisfactory response in the calorimeter. None of the nitrofurans were water soluble so prior to either MIC determination or incubation an accurately weighed sample of each compound was dissolved in 0.4ml DMSO and then 1.6ml $H_2O$ was added. When this solution was added to either tubes containing nutrient broth or to the fermentation vessels emulsions resulted. Good stirring minimised the problem in the calorimeter. No compensation was made for DMSO when diluting the samples as high concentrations of DMSO could falsify the results. Fig. 19 shows the structure-activity relationship of equimolar amounts of the four nitrofurans. Two types of responses were observed. A decrease in power output was observed for compounds 1 and 3 whereas an increase in power output was observed for compounds 2 and 4. No dependence of MIC on either RMM or power could be observed. Obviously a change in the side chain resulted in a high variation in antibacterial activity or metabolism. The least active compounds seem to be compounds 1 and 2. Compound 4 showed a similar type of response to that of neomycin to E.coli and it would therefore be indicated that compound 3 was the most active.

A real life example of issues relating to antibiotic treatment and their adverse effects that causes death (in an AIDS-like fashion, but would indicate stimulation of both prokaryotic and eukaryotic mRNA synthesis and consistency with what was observed during early Aids cases) is shown by the following case study.

An Animal case study

A domestic cat was given a mixture of antibiotics as eye drops in response to a scratch to the eye. The cat had a major adverse reaction which caused serious weight loss (that later caused its death). The cat was given 30 days of vancomycin treatment without any effect to the eye. Previously the cat had never been given milk (as many animals are allergic to cow's milk). The cat was now given full cream milk as part of a regimen to promote weight gain (the cat also continued to eat its usual amount of daily cat food throughout the period of weight loss). Initially weight was gained but within 24 hours weight loss resumed (presumably because the pathogenic bacteria/viruses had found a way of utilising lactose). An autopsy indicated bone-cancer as cause of death. The body wasting caused by the weight loss was very similar to that observed in early AIDS cases. However the cat had been regularly vaccinated against HTLV 1 through its 16 year life (and had never been ill in that period).

**Claims**

1. Method of determining the structure activity relationship or mode of action of a first and second compound comprising providing each of the compounds to a microorganism sample and detecting activity of each compound on the sample by detection of the heat generated in the sample using a calorimeter, thereby allowing any difference in activity to be correlated to difference in structure or mode of action between the two compounds.

2. Method according to claim 1 which comprises carrying out the method as defined in claim 1 to determine which compound has the greater activity and thus to determine which structural component is responsible for the increased activity, and then providing a third compound to a sample of said microorganism and detecting the activity of the third compound by detection of the heat generated in the sample using a calorimeter, wherein the third compound differs from the first and second compounds but comprises the structural component previously found to be responsible for increased activity, thereby allowing the identification of further structural components which may increase activity if the third compound has a greater activity than the first or second compounds, and optionally further carrying out detection of the activity of further compounds on a sample of the said microorganism to identify further structural features which cause increased activity.

3. Method according to claim 1 or 2 wherein any of the compounds which is analysed differs from any of the other compounds by virtue of any of the following groups: hydrogen, halogen, hydroxy, amino, thio, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ carbocyclyl, 5- to 10-membered heterocyclyl, -Het-L, -L-Het-L', -L-A, -Het-A or -Het-L-A, wherein L and L' are the same or different and represent $C_1$-$C_6$ alkyl or $C_2$-$C_6$ alkenyl, Het is O, S or NR, wherein R is hydrogen or $C_1$-$C_6$ alkyl, and A is a $C_6$-$C_{10}$ aryl, 5- to 10-membered heteroaryl, $C_3$-$C_6$ carbocyclyl or 5- to 10-membered heterocyclyl moiety.

4. Method according to any one of claims 1 to 3 wherein the compounds are peptides, proteins or antibodies which differ from each other in respect to:

   - amino acid sequence,
   - type or extent of glycosylation, or
   - type or extent of lipid modification.

5. Method according to any one of claims 1 to 4 further comprising dispensing the selected compound found to have the greater activity into a composition.

6. Method of testing the efficacy of a vaccine in an individual comprising adding in vitro the vaccine to a fluid or tissue sample from the individual and determining whether or not an immune response occurs in the sample to the vaccine by measuring heat output from the sample by a calorimeter.

7. Method of testing the efficacy of a vaccine in an individual comprising determination of the level and/or type of immune response by measuring heat output from a fluid or tissue sample taken from the individual at a defined time point after administration of the vaccine to the individual, wherein heat output is measured using a calorimeter.

8. Method according to claim 7 wherein heat output from two or more samples taken at successive time points after said administration is measured.

9. Method according to any one of claims 6 to 8 which further comprises measuring the levels of antibodies and/or T cells and/or macrophages and/or immune system modulators in the sample.

10. Method of testing whether or not a candidate substance has anti-viral activity comprising adding the candidate substance to a viral sample and detecting interaction of the substance with the virus by measuring the heat output from the sample by calorimetry.

11. Method according to claim 10 wherein cells capable of being infected by the virus are present in the sample.

12. Method according to claim 10 or 11 comprising determining whether or not a particular anti-viral compounds or combination of anti-viral compounds is suitable for administering to an individual with a viral condition comprising adding said compound or combination to a fluid or tissue sample from the individual and detecting inhibition of viral activity or growth by measuring heat output from the sample by means of a calorimeter.

13. Method according to any one of claims 10 to 12 wherein the virus is HIV, HSV, RSV or viruses which are members of the families Picornaviridae (e.g. polioviruses or rhinoviruses); Caliciviradae; Togaviridae (e.g. rubella virus or dengue virus); Flaviviridae; Coronaviridae; Reoviridae (e.g. rotavirus.); Birnaviridae; Rhabodoviridae (e.g. rabies virus); Orthomyxoviridae (e.g. influenza virus types A, B, and C); Filoviridae; Paramyxoviridae (e.g. mumps virus, measles virus, respiratory syncytial virus or parainfluenza virus); Bunyaviridae; Arenaviridae; Retroviradae (e.g. HTLV-I; or HTLV-II; Papillomavirus, the tick-bourne encephalitis viruses.

14. Method of determining a suitable administration regimens for a patient comprising taking a sample from a patient on a first administration regimen and detecting the effectiveness of the first administration regimen by measurement of the immune response, microorganism load or numbers of malignant cells using calorimetry, and then optionally if the first administration regimen is found to be of low effectiveness selecting a second administration regimen for the patient.

15. Method according to claim 14 wherein the first and second administration regimen differ in respect to the drug or combination of drugs which are administered.

16. Method according to claims 14 or 15 wherein the patient is infected with HIV.

17. Method of determining whether an anti-microorganism or anti-infective compound is able to cause growth of the microorganism/virus/mammalian cell comprising adding the compound to the microorganism, optionally at a pre-determined low concentration threshold, such as < 10xMIC, <8xMIC, < 2xMIC, < 1xMIC to determine whether or not growth or heat output of the microorganism/virus/mammalian cell occurs in response to the addition of the compound, wherein growth of the microorganism is optionally measured by detecting heat output from the microorganism/virus/mammalian cell using a calorimeter, and wherein the compound may be alone or in a combination with 2 or more other anti-microorganism or anti-infective agents.

18. Method according to claim 17 wherein if the said compound is found to cause increased growth of microorganisms at the pre-determined low concentration, then administering the compound to an individual suffering from a condition caused by the microorganism or at risk of such a condition, at a high dosage, but optionally not at dosages causing increased respiration of the said compound.

19. Method according to any one of the preceding claims wherein the individual, patient or eukaryote is a human, animal, mammal, primate, bird, domestic pet, agricultural animal, wild animal, sporting animal or fish or where the sample is from an individual of any such organism.

20. Method according to any one of the preceding claims wherein the individual, patient or eukaryote is of any of the following species, or where the sample is from an individual of any of the following species: horse, pig, cow, dog, cat, sheep, goat, turkey, chicken, camel, llama, deer, duck, fish, monkey, mouse, rat, guinea pig or hamster.

21. Method according to any one of the preceding claims wherein the calorimeter is able to detect changes in heat flow of 25 nanocalories/sec and/or heat effects of 10 microcalories.

**22.** Kit for carrying out a method according to any of the preceding claims comprising a calorimeter suitable for use in said method.

# Fig. 1

Interaction of Antibiotics with Bacillus Pumilus

Interaction of Antibiotics with Bacillus Pumilus 5 – 11 hours growth

Fig. 2

EP 1 785 721 A1

Legend:
—▲— Growth Curve Basillus Pumilus
——— Neomycin 15% incline 1ug/ml
—◆— Neomycin 10% incline 2ug/ml
- - - - Neomycin added at inoculation 0.5ug/ml
—■— Neomycin 25% incline 1ug/ml
— — Neomycin 10% incline 1ug/ml
—✕— Neomycin 10% incline 4ug/ml
—●— Neomycin added at inoculation

EP 1 785 721 A1

**Fig. 3**

Interaction of Antibiotics with Microccous Flavus

31

# FIG 4

FROZEN
Magnification x 43.000

FRESH
Magnification x 43.000

## Fig. 5

Bacitracin treated

Micrococcus flavus
(frozen)

0.025 x MIC

Bacitracin treated

Micrococcus flavus
(fresh)

0.025 x MIC

## Fig. 6

Interactions of AntiBiotics with Bordetella bronchiceptica

Legend:
- – ▲ – Growth Curve BB
- 2 ug/ml POL B added at 18% incline
- 0.7 ug/ml POLB added at 18% incline
- ◇ 1ug/MLPOLB added at 18% incline
- □ 0.35 ug/ml POLB added at 18% incline

Time (H)

## FIG 7

FROZEN
Magnification x 59000

FRESH
Magnification x 75000

## FIG 8

Polymyxin treated
Bord bronch (frozen)
1µg/ml
Magnification x 75000

Polymyxin treated
Bord bronch (fresh)
1µg/ml
Magnification x 59000

## Fig. 9

Interactions of AntiBiotic mixtures with Bordetella bronchiceptica

# Interactions of Antibiotic Mixtures with Micrococus Flavus

EP 1 785 721 A1

Fig. 10

## Fig. 11

Interaction of Antibiotic Mixtures with Bacillus Pumilus

**Fig. 12**

EM for Equimolar combinations against <u>Bordetella bronchiceptica</u>

EM for TRISEP combinations against <u>Bordetella bronchicptica</u>

## Fig. 13

EM for Equimolar
combinations against
Micrococcus flavus

EM for TRISEP
combinations against
Micrococcus flavus

Fig. 14

EM for Equimolar
combinations against
Bacillus pumilus

EM for TRISEP
combinations against
Bacillus pumilus

Interactions of Polymyxin B with Escherichia coli

uv heatoutpt

Time (min)

- ▲ - Respiration curve —□— 5ug/ml POLB —✕— 10ug/ml POLB
—○— 29 ug/ml POLB ——— 40 ug/ml POLB ———

Fig. 15

EP 1 785 721 A1

## Fig. 16

Interactions of Neomycin with Escherichia coli

## FIG 17

## Fig. 18

Interactions of Mixtures of Antibiotics with Escherichia coli

# Structure Activity Relationship of Nitro Furans Tested against respiring E.coli

Time (min)

- - ▲ - - Control —□— NF1 —✕— NF2 —○— NF3 ——— NF4 ———

EP 1 785 721 A1

Fig 19

## EUROPEAN SEARCH REPORT

**European Patent Office**

**Application Number**

EP 05 25 6971

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | US 3 765 237 A (BLACKMER D,US)<br>16 October 1973 (1973-10-16)<br>* column 1, lines 4-24 *<br><br>* column 2, lines 34-39 *<br>* column 5, lines 45-55 *<br>* abstract *<br>----- | 1,22<br><br>2-5,<br>19-21 | INV.<br>G01N25/20<br>G01N33/15<br>G01N33/48<br>G01K17/00 |
| X<br><br><br><br><br><br><br><br><br>A | YANG Y ET AL: "Microcalorimetry is a sensitive method for studying the effect of nucleotide mutation on promoter activity"<br>JOURNAL OF BIOCHEMICAL AND BIOPHYSICAL METHODS, AMSTERDAM, NL,<br>vol. 62, no. 3,<br>31 March 2005 (2005-03-31), pages 183-189,<br>XP004759805<br>ISSN: 0165-022X<br>* the whole document *<br><br>----- | 22<br><br><br><br><br><br><br><br><br>1-5,<br>19-21 | |
| X<br><br><br><br><br><br><br><br><br><br>A | ULLRICH F ET AL: "Performance of calorimetric methods for the investigation of microbial systems in combination with additional sensors"<br>ANAL. BIOANAL. CHEM.; ANALYTICAL AND BIOANALYTICAL CHEMISTRY NOVEMBER 2005,<br>vol. 383, no. 5, November 2005 (2005-11), pages 747-751, XP002366779<br>published online: 28 June 2005<br>* page 747, paragraphs ABSTRACT,INTRODUCTION *<br>* page 750, left-hand column, last paragraph - right-hand column, line 2 *<br>----- | 22<br><br><br><br><br><br><br><br><br><br>1-5,<br>19-21 | TECHNICAL FIELDS SEARCHED (IPC)<br><br>G01N<br>G01K |

-/--

~~The present search report has been drawn up for all claims~~

| Place of search | Date of completion of the search | Examiner |
|---|---|---|
| Munich | 9 February 2006 | Filipas, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 05 25 6971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/038227 A1 (VERWAERDE PHILIPPE ET AL) 26 February 2004 (2004-02-26) | 22 | |
| A | * paragraphs [0001], [0035] - [0037], [0040], [0083], [0084] * | 1-5, 19-21 | |
| X | US 6 193 413 B1 (LIEBERMAN DAVID S) 27 February 2001 (2001-02-27) | 22 | |
| A | * column 1, lines 15-26 * <br><br> * column 2, lines 38-60 * <br> * column 13, line 58 - column 14, line 19 * <br><br> * column 15, lines 6-13 * | 1-5, 19-21 | |
| X | PATENT ABSTRACTS OF JAPAN vol. 008, no. 193 (C-241), 5 September 1984 (1984-09-05) & JP 59 088098 A (SUNTORY KK), 21 May 1984 (1984-05-21) | 22 | |
| A | * abstract * | 1 | |

TECHNICAL FIELDS
SEARCHED      (IPC)

~~The present search report has been drawn up for all claims~~

| Place of search | Date of completion of the search | Examiner |
|---|---|---|
| Munich | 9 February 2006 | Filipas, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent

Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5, 19-22

50

European Patent
Office

**LACK OF UNITY OF INVENTION
SHEET B**

Application Number

EP 05 25 6971

The Search Division considers that the present European patent application does not comply with the
requirements of unity of invention and relates to several inventions or groups of inventions, namely:

1. claims: 1-5,19-22

    Method of determining the structure activity relationship or
    mode of action of a first and second component by detection
    of heat using a calorimeter,
    and
    Kit for carrying out said method, comprising a calorimeter.
    ---

2. claims: 6,9,19-22

    Method of testing the efficacy of a vaccine in an
    individual, comprising adding in vitro the vaccine to a
    fluid/tissue sample from the individual, by detection of
    heat using a calorimeter,
    and
    Kit for carrying out said method, comprising a calorimeter.
    ---

3. claims: 7-9,19-22

    Method of testing the efficacity of a vaccine in an
    individual by detection of heat using a calorimeter at a
    defined time point after administration of the vaccine to
    the individual,
    and
    Kit for carrying out said method, comprising a calorimeter.
    ---

4. claims: 10-13,19-22

    Method of testing whether or not a substance has anti-viral
    activity by detection of heat using a calorimeter,
    and
    Kit for carrying out said method, comprising a calorimeter.
    ---

5. claims: 14-16,19-22

    Method of determining a suitable administration regimen for
    a patient by using calorimetry,
    and
    Kit for carrying out said method, comprising a calorimeter.
    ---

6. claims: 17-22

**European Patent**
**Office**

**LACK OF UNITY OF INVENTION**
**SHEET B**

Application Number

EP 05 25 6971

The Search Division considers that the present European patent application does not comply with the requirements of unity of invention and relates to several inventions or groups of inventions, namely:

```
         Method of determining whether an anti-microorganism or
         anti-infective compound is able to cause growth of the
         microorganism/virus/mammalian cell,
         and
         Kit for carrying out said method, comprising a calorimeter.
                              ---
```

## ANNEX TO THE EUROPEAN SEARCH REPORT
## ON EUROPEAN PATENT APPLICATION NO.

EP 05 25 6971

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2006

| Patent document cited in search report | | Publication date | Patent family member(s) | | Publication date |
|---|---|---|---|---|---|
| US 3765237 | A | 16-10-1973 | DE | 2216411 A1 | 26-10-1972 |
| | | | FR | 2132484 A5 | 17-11-1972 |
| | | | GB | 1341944 A | 25-12-1973 |
| | | | IT | 972421 B | 20-05-1974 |
| | | | JP | 53025273 B | 26-07-1978 |
| US 2004038227 | A1 | 26-02-2004 | AU | 5996101 A | 20-11-2001 |
| | | | AU | 5996201 A | 20-11-2001 |
| | | | WO | 0185978 A2 | 15-11-2001 |
| | | | WO | 0185901 A2 | 15-11-2001 |
| | | | EP | 1281067 A2 | 05-02-2003 |
| | | | EP | 1281068 A2 | 05-02-2003 |
| | | | JP | 2003532876 T | 05-11-2003 |
| | | | JP | 2003532877 T | 05-11-2003 |
| | | | US | 2004038228 A1 | 26-02-2004 |
| US 6193413 | B1 | 27-02-2001 | EP | 1194766 A1 | 10-04-2002 |
| | | | WO | 0079254 A1 | 28-12-2000 |
| JP 59088098 | A | 21-05-1984 | NONE | | |

For more details about this annex : see Official Journal of the European Patent Office, No. 12/82